# EUROPEAN PATENT SPECIFICATION

(11) **EP 2 917 374 B1**
(45) Date of publication and mention of the grant of the patent: **27.03.2019**
(21) Application number: 13853653.7
(22) Date of filing: 06.11.2013
(51) Int. Cl.: G01N 33/574, C12Q 1/68, A61K 31/52, A61K 31/7076

(54) **TECHNIQUES FOR TREATING ACUTE LYMPHOBLASTIC LEUKEMIA (ALL)**
VERFAHREN ZUR BEHANDLUNG VON AKUTER LYMPHOBLASTISCHER LEUKÄMIE
TECHNIQUES POUR TRAITER LA LEUCÉMIE AIGÜE LYMPHOBLASTIQUE (LAL)

(30) Priority: 06.11.2012 US 201261723315 P
(43) Date of publication of application: 16.09.2015
(73) Proprietor: The Trustees of Columbia University in the City of New York, New York, NY 10027 (US)
(72) Inventor: FERRANDO, Adolfo, New York, NY 10025 (US); RABADAN, Raul, New York, NY 10027 (US); PALOMERO, Teresa, New York, NY 10025 (US)
(74) Representative: Chapman, Paul Gilmour
(86) International application number: PCT/US2013/068821
(87) International publication number: WO 2014/074651

(56) References cited:
- WO-A2-2013/169771
- US-A1- 2010 311 606
- US-A1- 2011 183 864
- Julia Meyer ET AL: "Relapse-specific mutations in cytosolic 5'-nucleotidase II in childhood acute lymphoblastic leukemia", Journal of Clinical Oncology, 2012 ASCO Annual Meeting Abstracts., 17 May 2012 (2012-05-17), XP055271165, Retrieved from the Internet: URL:http://hwmaint.meeting.ascopubs.org/cg i/content/abstract/30/15_suppl/9507 [retrieved on 2016-05-09]
- FANG LI ET AL: "Ecto-5'-Nucleotidase and Thiopurine Cellular Circulation: Association with Cytotoxicity", DRUG METABOLISM AND DISPOSITION, vol. 38, no. 12, 20 September 2010 (2010-09-20), pages 2329-2338, XP055271283, US ISSN: 0090-9556, DOI: 10.1124/dmd.110.035220
- JORDHEIM L ET AL: "Drug resistance to cytotoxic nucleoside analogues.", CURRENT DRUG TARGETS, vol. 4, no. 6, August 2003 (2003-08), pages 443-460, XP009189992, ISSN: 1389-4501
- N. GOKBUGET ET AL: "High single-drug activity of nelarabine in relapsed T-lymphoblastic leukemia/lymphoma offers curative option with subsequent stem cell transplantation", BLOOD, vol. 118, no. 13, 28 June 2011 (2011-06-28), pages 3504-3511, XP055271617, US ISSN: 0006-4971, DOI: 10.1182/blood-2011-01-329441
- PIETERS ET AL.: 'Relation of 5'-nucleotidase and phosphatase activities with immunophenotype, drug resistance and clinical prognosis in childhood leukemia' LEUKEMIA RESEARCH vol. 16, no. 9, 01 September 1992, pages 873 - 880, XP026290593
- MITRA ET AL.: 'Genetic Variants in Cytosolic 5'-Nucleotidase II Are Associated with Its Expression and Cytarabine Sensitivity in HapMap Cell Lines and in Patients with Acute Myloid Leukemia' THE JOUMAL OF PHARMACOLOGY AND EXPERIMENTAL THERAPEUTICS vol. 339, no. 1, 28 June 2011, pages 9 - 23, XP055254190
- BROUWER ET AL.: 'Role of 5'-nucleotidase in thiopurine metabolism: Enzyme kinetic profile and association with thio-GMP levels in patients with acute lymphoblastic leukemia during 6-mercaptopurine treatment' CLINICA CHIMICA ACTA vol. 361, 28 June 2005, pages 95 - 103, XP005092826
- GALMARINI ET AL.: 'Role of IMP-selective 50-Nucleotidase (cN-II) in Hematological Malighancies' LEUKEMIA & LYMPHOMA vol. 44, 01 July 2003, pages 1105 - 1111, XP008179210
- TZONEVA ET AL.: 'Activating mutations in the NT5C2 nucleotidase gene drive chemotherapy resistance in relapsed ALL' NATURE MEDICINE vol. 19, no. 3, 03 February 2013, pages 368 - 373, XP055254199
- ASTER ET AL.: 'Resistance revealed in acute lymphoblastic leukemia' NATURE MEDICINE vol. 19, no. 3, 06 March 2013, pages 264 - 265, XP055254203
- MEYER ET AL.: 'Screening for gene mutations: will identification of NT5C2 mutations help predict the chance of relapse in acute lymphoblastic leukemia?' EXPERT REVIEW OF HEMATOLOGY vol. 6, no. 3, 01 June 2013, pages 223 - 224, XP008179211

## Description

### BACKGROUND OF THE INVENTION

Acute Lymphoblastic Leukemia (ALL) is an aggressive hematological tumor resulting from the malignant transformation of hematopoietic lymphoid progenitors. ALL develops from early forms of lymphocytes. It can start in either early B lymphocytes (B cells) or T lymphocytes (T cells) at different stages of maturity. ALL starting in T-cells is called T-cell ALL and abbreviated T-ALL; similarly, ALL starting in B-cells is called B-cell ALL and abbreviated B-ALL. Conventional chemotherapy treatment includes nucleoside therapy, for example with a combination of 6-mercaptopurine (6-MP) and 6-thioguanine (6-TG). However, despite intensive chemotherapy, 20% of pediatric and over 50% of adult T-cell acute lymphoblastic leukemia (T-ALL) patients show transient responses to treatment and ultimately die of their disease.

Meyer et al (J Clin Oncology, 2012 ASCO Ann Meeting Abs vol 30, No 15 suppl, 2012: p9507) describes relapse-specific mutations in cytosolic 5'-nucleotidase II (NT5C2) in childhood acute lymphoblastic leukemia

### SUMMARY OF THE INVENTION

Various embodiments are based on the discovery of certain new somatic single nucleotide substitution mutations of the gene for protein *NT5C2* (*NT5C2* gene variants) found in some ALL patients. The mutated proteins encoded by these *NT5C2* gene variants were discovered to inactivate at least some nucleoside analogs typically used for therapy of ALL. Certain embodiments are directed to determining treatment efficacy based on detecting these newly discovered *NT5C2* gene variants, listed below. These new *NT5C2* gene variants can be used to diagnose ALL patients who have relapsed or who are at high risk of relapsing, and to determine that a subject whose leukemia cells have the variant will not respond to conventional nucleoside analog therapy, as described below, but benefit from a revised treatment protocol. In a first aspect there is provided a nucleoside analog selected from 6-mercaptopurine, 6-thioguanine and nelarabine for use in a method of treating of a subject with acute lymphoblastic leukemia, wherein
(a) if the subject does not have the presence of a detectable *NT5C2* gene mutation which results in an associated protein mutation selected from the group consisting of R238W, R238L, R291W, K359Q, S360P, R367Q, L375F, D407A, D407Y and Q523*, the nucleoside analog is 6-mercaptopurine or 6-thioguanine; and
(b) if the subject has the presence of a detectable *NT5C2* gene mutation which results in an associated protein mutation selected from the group consisting of R238W, R238L, R291W, K359Q, S360P, R367Q, L375F, D407A, D407Y and Q523*, the nucleoside analog is nelarabine.

In various embodiments, the subject is an animal, typically a mammal, preferably a human, and the biological sample is selected from the group consisting of hair, nails, serum, blood, peripheral blood, plasma, sputum, saliva, mucosal scraping, urine, pleural effusion fluid, cerebrospinal fluid, bone marrow or tissue biopsy. The preferred source is bone marrow or peripheral blood because leukemia cells are primarily recovered from blood and bone marrow aspirates. In various other embodiments, a preferred source is cerebrospinal fluid, pleural effusions and tissue biopsy which are currently often analyzed for leukemia.

This disclosure teachesobtaining a biological sample taken from a subject having ALL, and detecting the presence or absence in the biological sample of a biomarker selected from the group consisting of an *NT5C2* gene mutation, an mRNA transcribed from the *NT5C2* gene mutation, or a protein encoded by the *NT5C2* gene mutation. The disclosure also teaches determining that the subject has increased resistance to treatment with 6-mercaptopurine or 6-thioguanine, if the biomarker is detected. In some embodiments, the disclosure still further teaches treating the subject with a nucleoside analog other than 6-mercaptopurine or 6-thioguanine if the biomarker is detected.

In a second set of embodiments, a kit is provided for detecting in a biological sample the presence of a biomarker in a biological sample selected from the group consisting of a *NT5C2* gene mutation, an mRNA transcribed by the *NT5C2* gene mutation, or a protein encoded by the *NT5C2* gene mutation. The kit includes reagents for detecting the presence of the *NT5C2* gene mutation, the mRNA transcribed from the *NT5C2* gene mutation, or the protein encoded by the *NT5C2* gene mutation and instructions for use of the kit to determine if a subject having Acute Lymphoblastic Leukemia has reduced sensitivity to treatment with 6-mercaptopurine or 6-thioguanine.

In particular there is provided a kit for detecting the presence in a biological sample the presence of a detectable *NT5C2* gene mutation which results in an associated protein mutation selected from the group consisting of R238W, R238L, R291W, K359Q, S360P, R367Q, L375F, D407A, D407Y and Q523*, the kit comprising reagents for detecting the presence of the *NT5C2* gene mutation, and instructions for use of the kit.

This disclosure further teachesa screening assay for identifying a ligand that binds with high affinity to *NT5C2* protein or to a biologically active fragment or variant thereof. The assay includes screening a ligand library against *NT5C2* or a biologically active fragment or variant thereof to identify a ligand that binds with high affinity to the *NT5C2* protein and isolating the high affinity ligand from the library. The assay further includes combining the high affinity ligand, the *NT5C2* protein and the nucleoside analog under conditions that permit the *NT5C2* protein to bind to the nucleoside analog, and determining if the *NT5C2* protein binds to the nucleoside analog in a mixture forming a *NT5C2* protein-nucleoside complex. The assay still further includes selecting and identifying the high affinity ligand as one that prevents *NT5C2* protein from inactivating the nucleoside analog, if the *NT5C2* protein-nucleoside complex is not formed.

This disclosure further teachesa pharmaceutical composition comprising a high affinity *NT5C2* binding ligand identified using any of the described screening methods.

This disclosure further teaches anallele-specific PCR assay directed to one of the *NT5C2* gene variants.

Still other aspects, features, and advantages of the invention are readily apparent from the following detailed description, simply by illustrating a number of particular embodiments and implementations, including the best mode contemplated for carrying out the invention. Accordingly, the drawings and description are to be regarded as illustrative in nature, and not as restrictive.

### BRIEF DESCRIPTION OF THE DRAWINGS

The present invention is illustrated by way of example, and not by way of limitation, in the figures of the accompanying drawings and in which like reference numerals refer to similar elements and in which:
FIG. 1 is a block diagram that illustrates example activity of the *NT5C2* protein;
FIG. 2A and FIG. 2B form a block diagram that illustrates example locations of mutations on the *NT5C2* protein observed in patients suffering relapsed pediatric T-ALL, according to an embodiment;
FIG. 3A through FIG. 3F are deoxyribonucleic acid (DNA) sequencing chromatograms that illustrate example paired diagnosis and relapse genomic T-ALL DNA samples showing relapse specific heterozygous *NT5C2* mutations, according to an embodiment;
FIG. 4 is a flow chart that illustrates an example method for detecting or treating an ALL patient at risk of relapse during conventional nucleoside analog treatment; according to an embodiment;
FIG. 5A is a diagram of the *NT5C2* protein with a location of the *K359Q* mutation and binding sites for ATP and IMP, according to some embodiments;
FIG. 5B through FIG. 5E are diagrams showing the relative locations on the *NT5C2* protein of other amino acids and IMP and ATP binding sites; according to various embodiments;
FIG. 6A is a bar graph that illustrates example increased activity in three *NT5C2* mutant proteins compared to wild type, according to an embodiment;
FIG. 6B through FIG. 6D are graphs that illustrate increased activity and less dependence on ATP concentration for at least six of seven *NT5C2* mutant proteins compared to wild type, according to various embodiments;
FIG. 6E is a table that illustrates less dependence of activity on ATP concentration for seven *NT5C2* mutant proteins compared to wild type, according to various embodiments;
FIG. 7A is a diagram of the nucleoside analog 6-mercaptopurine (6-MP);
FIG. 7B is a table that illustrates higher resistance of cells expressing three *NT5C2* mutant proteins in the presence of 6-MP in two cell lines, according to various embodiments;
FIG. 7C through FIG. 7H are graphs that illustrate example higher viability of cells expressing three *NT5C2* mutant proteins, respectively, in the presence of various doses of 6-MP in two cell lines, respectively, according to various embodiments;
FIG. 8A is a diagram of the nucleoside analog 6-thioguanine (6-TG);
FIG. 8B is a table that illustrates higher resistance of cells expressing three *NT5C2* mutant proteins in the presence of 6-TG in two cell lines, according to various embodiments;
FIG. 8C through FIG. 8H are graphs that illustrate example higher viability of cells expressing three *NT5C2* mutant proteins, respectively, in the presence of various doses of 6-TG in two cell lines, respectively, according to various embodiments;
FIG. 9 is a table that illustrates an example distribution of mutant *NT5C2* proteins in patients who relapse while on conventional treatment, with 100% of such patients expressing a mutant *NT5C2* protein, according to an embodiment;
FIG. 10A is a diagram of the nucleoside analog nelarabine;
FIG. 10B through FIG. 10D are graphs that illustrate example similar viability of cells expressing a control protein and three *NT5C2* mutant proteins, respectively, in the presence of various doses of nelarabine, according to various embodiments;
FIG. 11 is a diagram that illustrates an example model for ALL relapse during treatment in the presence of *NT5C2* mutant proteins, according to an embodiment;
FIG. 12 is a table that illustrates an example data finding no *NT5C2* mutations in ALL patients who are originally diagnosed with the disease or in remission, according to an embodiment;
FIG. 13A is a diagram that illustrates an example allele-specific polymerase chain reaction (PCR) with blocker for wild types used to detect genetic variant mutations for *NT5C2,* according to an embodiment;
FIG. 13B is a graph that illustrates an example amplification of a genetic variant for *NT5C2* using the allele specific PCR process of FIG. 13A, according to an embodiment;
FIG. 13C and FIG. 13D are graphs that illustrate example calibration and sensitivity, respectively, of one genetic variant mutation for *NT5C2* using the allele-specific PCR process of FIG. 13A, according to an embodiment.

### DETAILED DESCRIPTION

### Definitions

"Biomarker" as used herein means a genetic variant, of any size or type, contained within an individual's genome that is associated with a disease or condition, such as drug response, or any molecule derived therefrom, including a DNA molecule expressing the genetic variant, an RNA molecule transcribing the genetic variant, or a polypeptide that the genetic variant encodes.

"Genetic variation" as used herein means genotypic differences among individuals in a population, at one or more polymorphic loci, excluding common (wild) types, and including an insertion of one or more nucleotides, a deletion of one or more nucleotides, one or more single nucleotide sequence variations (SNVs), such as SNPs, copy number variation, such as one or more duplications, sequence rearrangements, such as one or more inversions, one or more translocations, or one or more repeat sequence expansions or contractions (*i.e*., changes in microsatellite sequences) at one or more polymorphic loci of interest within a target region of interest as compared to known reference sequences. Gene variants such as SNPs and SNVs can alter gene structure and/or expression level, and thus, in many cases, protein structure and/or expression level.

"Somatic cell mutation" or "somatic mutation" means a mutation in a somatic (non germ line cell) that is acquired. Somatic cells are diploid, containing two copies of each chromosome, whereas the germ cells are haploid, as they only contain one copy of each chromosome. The changes/mutations in *NT5C2* occur in the leukemia cells during the process of leukemia formation. They are not present in the population as a whole or in the patient at birth, and they are not present in any other tissues of the patient other than the leukemia cells.

"High affinity" in the context of binding of a ligand to *NT5C2* protein refers to affinity, which reflects stability of binding. High affinity binding means that the ligand and the protein bind to each other tightly. Binding affinity can be defined quantitatively by dissociation constant.

"Ligand" as used herein means a substance (usually a small molecule, peptide or antibody or fragment thereof), that forms a complex by binding with high affinity to a target, such as the *NT5C2* protein. The binding occurs by intermolecular forces, such as ionic bonds, hydrogen bonds and van der Waals forces. Actual irreversible covalent binding between a ligand and a protein is rare in biological systems.

"Normal" as used herein, describes what is standard or the usual state. As applied in biology and medicine, a "normal state" or "normal person" is what is usual or most commonly observed. For example, individuals with disease are not typically considered normal. Example usage of the term includes, but is not limited to, "normal subject," "normal individual," "normal organism," "normal cohort," "normal group," and "normal population." A normal gene is also called a wild type (*WT*).

*"NT5C2* protein" as used herein is also known as "cystosolic purine 5'-nucleotodiase" and is a glycoprotein enzyme present in various organs and in many cells. The amino acid sequence is given by NP_036361.1 and NP_001127845.1, which are identical]. The enzyme catalyzes the hydrolysis of a 5'-ribonucleotide to a ribonucleoside and orthophosphate in the presence of water. It is cation-dependent and exists in membrane-bound and soluble forms. Novus Biologicals of Littleton, Colo., offers *NT5C2* peptides and *NT5C2* proteins for use in common research applications: ELISA, Protein Array, SDS-Page, and Western Blot. *NT5C2* Peptides and *NT5C2* Proteins can be used in a variety of model species, including human. To distinguish mutations of this protein, the wild type *NT5C2* protein is also designated with a suffix "*WT*," e.g., *"NT5C2 WT."* Various mutations are designated by *NT5C2* followed by a different suffix that indicates the amino acid substitution of the mutation. For convenience, in the following, the term *NT5C2* suffix is used to refer to the protein or the gene that encodes the protein, as will be clear by the context.

*"NT5C2* gene" means the gene that encodes for the *NT5C2* protein and has the official name "5'-nucleotidase, cytosolic II." The nucleotide sequence is given by D38524.1. This gene encodes a hydrolase that serves as an important role in cellular purine metabolism by acting primarily on inosine 5'-monophosphate and other purine nucleotides. To distinguish mutations of this gene, the wild type gene is also designated with a suffix "*WT*," e.g., *"NT5C2 WT."* Various mutations are designated by *NT5C2* followed by a different suffix that indicates the amino acid substitution resulting from the mutation. For convenience, in the following the term *NT5C2* suffix is used to refer to the protein or the gene that encodes the protein, as will be clear by the context.

"Nucleoside analogues" as used herein mean a range of antiviral and anticancer products used to prevent viral replication in infected cells and to treat cancer. Commonly used antitumor nucleoside analogs include 6-mercaptopurine, thioguanine, AraC, nelarabine and fludarabine among others.

The terms "polypeptide", "peptide", and "protein" are used interchangeably herein to mean a polymer of amino acid residues. The terms apply to amino acid polymers in which one or more amino acid residue is a modified residue, or a non-naturally occurring residue, such as an artificial chemical mimetic of a corresponding naturally occurring amino acid, as well as to naturally occurring amino acid polymers.

"Single nucleotide variant" or "SNV" as used herein means a DNA base within an established nucleotide sequence that differs from the known reference sequences. SNVs may be found within a patient sample (e.g., a cancer cell such as ALL), and may or may not be present in unperturbed populations, and include naturally occurring single nucleotide polymorphisms.

"Single nucleotide polymorphism" or "SNP" as used herein means a single nucleotide position in a genomic sequence for which two or more alternative alleles are present at an appreciable frequency (*e.g.,* at least 1%) in a population of organisms.

"Subject" as used herein means an object of a method or material, including mammals, e.g., humans, dogs, cows, horses, kangaroos, pigs, sheep, goats, cats, mice, rabbits, rats, and transgenic non-human animals. Synonyms used herein include "patient" and "animal."

"ALL" as used herein means Acute Lymphoblastic Leukemia, an aggressive hematological tumor resulting from the malignant transformation of lymphoid progenitors.

By *"NT5C2* gene variant" is meant a gene variant described herein and listed in Table 1, of which variants at least some mutations are associated with ALL relapse. A specific gene variant is indicated herein by a suffix that indicates a location in the amino acid sequence for the *NT5C2* protein where a genetic mutation has led to an amino acid substitution, as described in more detail below. The normal (wild type) gene is indicated by the suffix "*WT*." For convenience, the term *NT5C2* and suffix is used to refer to the protein or the gene that encodes the protein, as will be clear by the context.

### Overview

Treatment of ALL that fails to achieve complete remission, or allows relapse, after intensified chemotherapy is almost invariably associated with therapy resistance, disease progression and death. However, the specific mechanisms responsible for relapse and chemotherapy resistance in ALL was largely unknown before this current work. Using whole exome sequencing, multiple new activating mutations in the gene encoding *NT5C2,* an enzyme responsible for inactivation of nucleoside analog chemotherapy, have been identified in relapsing pediatric ALL patients at the time of relapse. This discovery supports a prominent role for enhanced nucleoside analog metabolism in disease progression and chemotherapy resistance in ALL. In addition, numerous new genes not previously implicated in the pathogenesis of ALL were identified, including three genes mutated at the time of relapse: *TP53* (*TP53 R213Q*), *BANP* (*BANP H391Y*) and *RPL11* (*RPL11 R18P*). Subsequent analysis identified two additional somatic *RPL11* mutant alleles; one (*RPL11 X178Q*) was present both at diagnosis and relapse; and one (*RPL11 G30fs*) specifically mutated at relapse.

Based on these and other discoveries described below, certain embodiments (also described in more detail in the Summary of the Invention) are directed to the newly identified mutations of *NT5C2,* and to methods for identifying subjects at risk of relapsing with ALL or who have relapsed, and subjects who are resistant to nucleoside therapy based on the presence of an *NT5C2* gene variant in leukemia cells from the subject.

Data concerning survival for patients with ALL in different treatment eras indicates low probability (about 10%) of survival even to 5 years, in the era from 1962 to 1966 and increasing survival in more recent eras 1967-1979, 1979-1983, 1984-1991, 1991-1999, and 2000-2007, respectively. While probability of survival has increased dramatically, there is still about a 20% chance of not surviving 5 years.

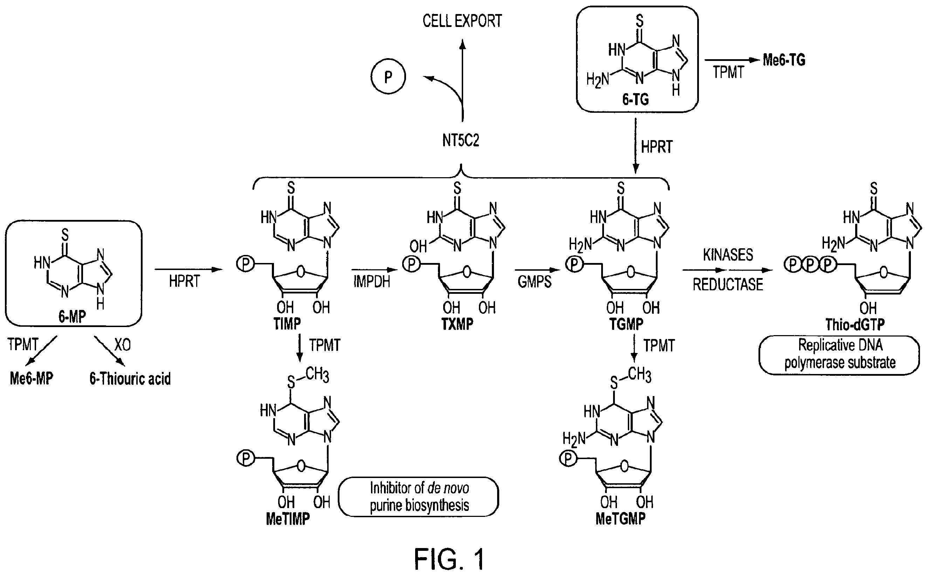

*NT5C2* is a ubiquitous enzyme responsible for the final dephosphorylation of 6-hydroxypurine nucleotide monophosphates such as IMP, dIMP, GMP, dGMP and XMP before they can be exported out of the cell (Oka, 1994; Spychala, 1988). In addition, and most notably, *NT5C2* can also dephosphorylate and inactivate 6-thioinositol monophosphate and 6- thioguanosine monophosphate which mediate the cytotoxic effects of 6-mercaptopurine (6-MP) and 6-thioguanine (6-TG) (Brouwer, 2005), two nucleoside analogs commonly used in the treatment of ALL. FIG. 1 is a block diagram that illustrates example activity of the *NT5C2* protein. FIG. 1 indicates that when nucleoside analog 6-mercaptopurine (6-MP) is introduced into an ALL cell, it is metabolized into THio-IMP (TIMP) then MeTIMP which inhibits purine biosynthesis, leading to cell death. In addition, TIMP metabolizes to Thio-XMP (TXMP) and then Thio-GMP (TGMP). If nucleoside analog 6-thioguanine (6-TG) is introduced into the cell, it is metabolized into with TGMP. TGMP can be converted to Thio-GTP with serves as substrate for RNA polymerase, thus interfering with RNA metabolism; and to to produce Thio-dGTP which serves as a substrate for replicative DNA polymerase, thus interfering with cell division. Thus a combination therapy involving 6-MP and 6-TG is common for treatment of subjects having ALL.

The normal (wild type) *NT5C2* protein dephosphorylates and inactivates the active metabolites of 6-MP and 6-TG, and acts to expel these metabolites TIMP, TXMP and TGMP fueled by available ATP. At sufficient doses of 6-MP and 6-TG, the available *NT5C2* and ATP cannot keep pace with the deleterious effects of the nucleoside analogs and the ALL cell line dies out.

The human gene for *NT5C2* is located on chromosome 10, starting at 104,845,940 base pairs (bp) from phosphotriesteras (pter) and ending at 104,953,056 bp from pter, comprising 107,117 bases. It is oriented on the minus strand and its sequence is cataloged at EnsEMBL Gene ID: ENSG00000076685. The NTFC2 gene (cloned gene GenBank accession number P49902 for NT5C2 protein Uniprot/Swiss-Prot ID; mRNA GenBank accession numbers NM001134373.2; and protein GenBank accession numbers NP001127845.1 in humans or 5'-nucleotidase, cytosolic II) is also known as GMP, NT5B, PNT5, and cN-11.

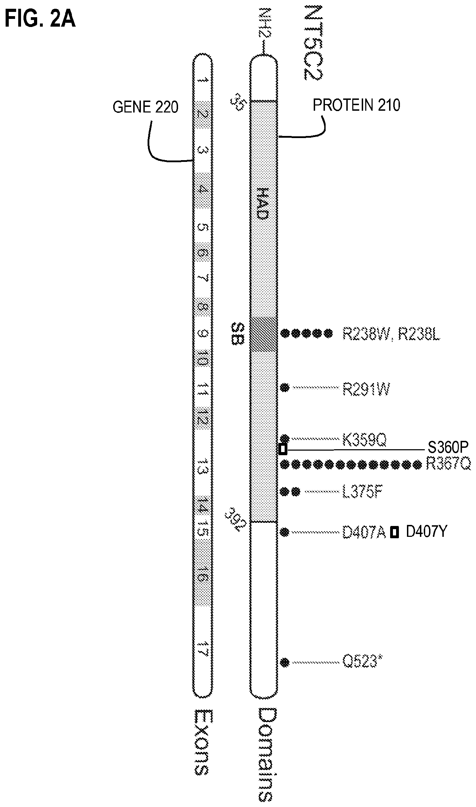

FIG. 2A is a block diagram that illustrates example locations of mutations on the *NT5C2* protein observed in patients suffering relapsed pediatric T-ALL, according to an embodiment. It was discovered, as described in more detail below, that these mutations cause enhanced *NT5C2* activity, with less dependence on ATP concentrations. FIG. 2A lines up the *NT5C2* protein 210 with the exons on the *NT5C2* gene 220 that codes for the protein 210. The protein 210 comprises several domains including a haloacid dehalogenase (HAD) domain from position 35 to position 392 surrounding a substrate binding (SB) domain. The *NT5C2* gene 220 includes 17 exons.

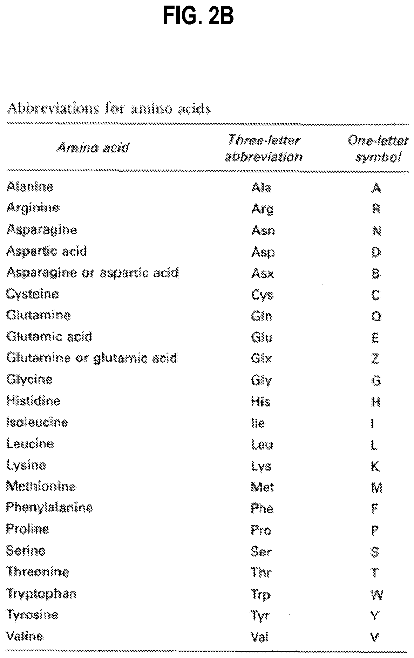

Mutations are designated herein, as is standard in the art, by the amino acid change to the *NT5C2 WT* protein in the format of letter-number-letter, where the number indicates the position of the amino acid in the protein from the end translated by the first exon, the first letter is the one letter symbol for the amino acid in the wild type of the protein at that location, and the second letter is the one letter symbol for the amino acid in the mutated protein at that location. For convenience, the one letter symbols for amino acids are listed in FIG. 2B. An asterisk indicates the data indicating the mutation creates a stop codon. Filled circles represent heterozygous missense mutations. Eight such missense mutations in the *NT5C2* protein were identified in relapsed pediatric samples and are shown. Multiple circles in the same amino acid position account for multiple patients with the same variant. The patient population includes 138 relapsed ALL patients, including 35 with B-ALL. Additional mutations determined from published data of patients outside the study are shown as open squares.

Each patient in the study is identified by a type of ALL (e.g., T-ALL) and a sequential number within the type. DNA from leukemic ALL blasts at diagnosis and at relapse and matched remission lymphocytes were provided by the Hemato-Oncology Laboratory, Department of Pediatrics, University of Padua, Padua, Italy. All samples were collected in clinical trials with informed consent and under the supervision of local ethics Institutional Review Board (IRB) committees. Consent was obtained from all patients at trial entry according to the Declaration of Helsinki. As will be shown in more detail below, every patient with at least one of the *NT5C2* mutations relapsed while being treated with conventional nucleoside analog therapy.

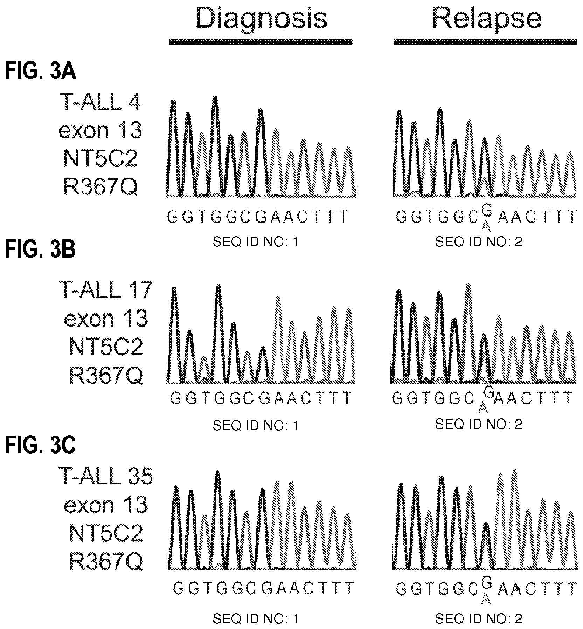

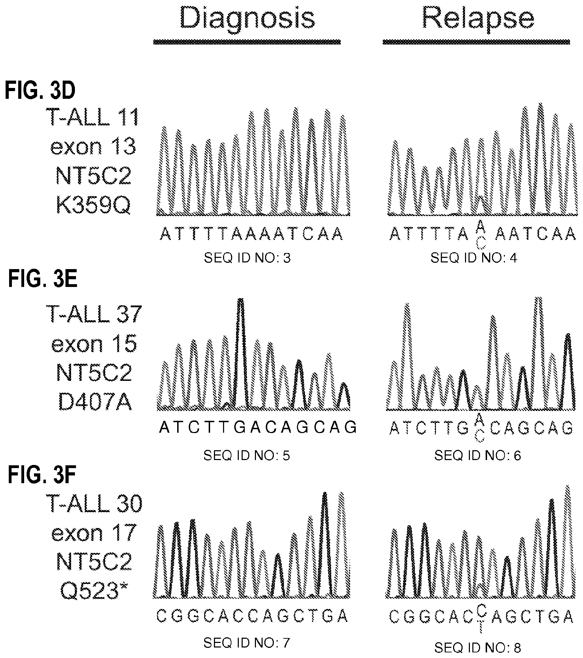

FIG. 3A through FIG. 3F are DNA sequencing chromatograms that illustrate example paired diagnosis and relapse genomic T-ALL DNA samples showing relapse specific heterozygous *NT5C2* mutations, according to an embodiment. Each DNA sequence is associated with one patient and shows one mutation between diagnosis and relapse. The patient identifier, exon identifier and mutation are specified on the left of each chromatogram, the nucleic acid base letter sequence and associated signal strength as a peak height is shown for each base in a unique 15 base portion of the exon. The standard base symbols are used: guanine (G), cytosine (C), adenine (A), and thymine (T). FIG. 3A (SEQ ID NOS: 1 and 2) shows that T-ALL patient 4 underwent an *R367Q* mutation associated with exon 13 involving a SNP that changes a G to A in the middle of this unique portion that covers the complete sequence of the exon. The 15 nucleotide segment shown is unique and defines the position of the SNP in the NT5C2 sequence. FIG. 3B and FIG. 3C (SEQ ID NOS: 1 and 2) show that each of T-ALL patients 17 and 35, respectively, also underwent an *R367Q* mutation associated with exon 13 involving the same SNP. FIG. 3D (SEQ ID NOS: 3 and 4) shows that T-ALL patient 11 underwent a *K359Q* mutation associated with exon 13 involving a SNP that changes a G to A in the middle of this unique 15 base portion of the exon. FIG. 3E (SEQ ID NOS: 5 and 6) shows that T-ALL patient 37 underwent a *D407A* mutation associated with exon 15 involving a SNP that changes an A to C. FIG. 3F (SEQ ID NOS: 7 and 8) shows that T-ALL patient 30 underwent a Q523* mutation associated with exon 17 involving a SNP that changes a C to T to form a stop codon. Each *NT5C2* protein mutation and associated exon and single nucleotide polymorphism (SNP) is listed in Table 1. Two mutations, detected in published data but not among the patient population of the rest of this study, are also included in table 1. These are *S360P* and *D407Y.*

**Table 1. NT5C2 Protein mutations and associated exons and SNPs in the NT5C2 gene**

| **Exon** | **Associated Protein mutation** | **SNP** |
|---|---|---|
| 9 | R238W | 712C>T |
| 9 | R238L | 713G>T |
| 11 | R291W | 871C>T |
| 13 | K359Q | 1075A>C |
| 13 | S360P | 1078T>C |
| 13 | R367Q | 1100G>A |
| 13 | L375F | 1123C>T |
| 15 | D407Y | 1219G>T |
| 15 | D407A | 1220A>C |
| 17 | Q523* | 1567C>T |

All amino acid mutations are based on the NT5C2 wild type protein sequence identified as NP_036361.1. All SNPs are based on the NT5C2 gene wild type sequence identified as D38524.1.

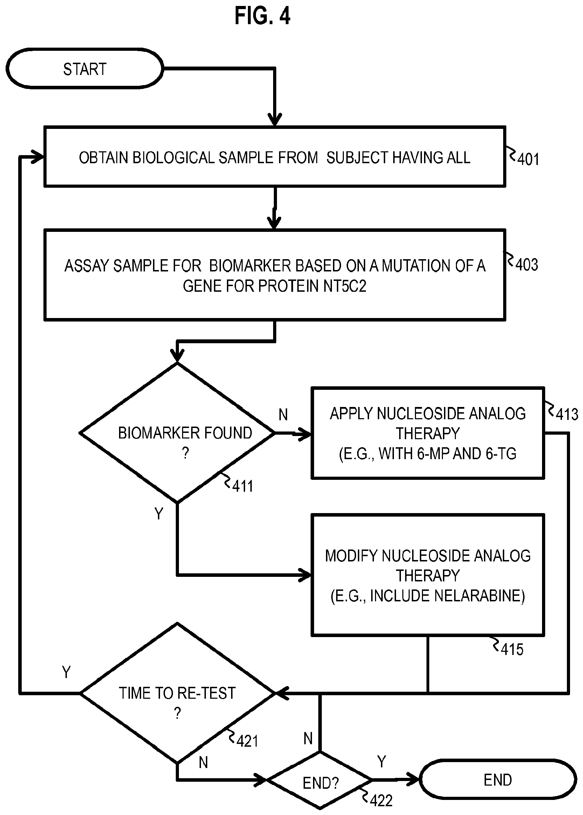

FIG. 4 is a flow chart that illustrates an example method for detecting or treating an ALL patient at risk of relapse from conventional nucleoside analog treatment; according to an embodiment. Although steps are depicted in FIG. 4 as integral blocks in a particular order for purposes of illustration, in other embodiments, one or more steps, or portions thereof, are performed in a different order, or overlapping in time, performed in series or in parallel, or are omitted, or one or more additional steps are included. For example, in some embodiments, the sample is collected and an assay is performed by others, and an individual simply performs steps 411 and following. In other embodiments, only the assay is performed.

In step 401 a sample from a subject having ALL is obtained. Any method may be used to obtain the sample, from drawing or excising the sample from the subject, to retrieving the sample from a cooler, freezer or other storage vessel, to receiving data about the sample. As described above, in various embodiments, the subject is an animal, typically a mammal, preferably a human, and the biological sample is selected from the group consisting of serum, blood, peripheral blood, plasma, bood cells, pleural effusion, cerebrospinal fluid, bone marrow or tissue biopsy. The preferred source is bone marrow or peripheral blood.

In step 403, an assay is performed to detect a biomarker based on a mutation of a gene for protein *NT5C2.* Recall that a biomarker refers to a DNA molecule expressing a genetic variant associated with a particular disease or condition, such as ALL relapse, an RNA molecule transcribing the genetic variant, or a polypeptide that the genetic variant encodes. For example, in various embodiments, an assay is performed for the DNA sequence, or for the messenger RNA, or for the protein for one or more of the mutations indicated in Table 1. In various embodiments, step 403 includes performing at least one of radioactive or fluorescent DNA sequencing, polymerase chain reaction (PCR), reverse transcription PCR(RTPCR), competitive allele-specific TaqMan® (Life Technologies, New York) PCR, allele-specific restriction-endonuclease cleavage, mismatch-repair detection, binding of MutS protein, denaturing-gradient gel electrophoresis, single-strand-conformation polymorphism detection, RNAase cleavage at mismatched base-pairs, chemical or enzymatic cleavage of heteroduplex DNA, methods based on oligonucleotide-specific primer extension, genetic bit analysis, oligonucleotide-ligation assay, oligonucleotide-specific ligation chain reaction (LCR), gap-LCR, peptide nucleic acid (PNA) assays, Southern Blot analyses, single stranded conformational polymorphism analyses (SSCP), deoxyribonucleic acid sequencing, restriction fragment length polymorphism (RFLP) analysis.

In some embodiments, step 403 includes one or more of assaying for mRNA transcribed from the mutated *NT5C2* gene detected using a method from the group comprising Northern blot analysis, nuclease protection assays (NPA), in situ hybridization, and reverse transcription-polymerase chain reaction (RT-PCR) mRNA sequencing.

In some embodiments, step 403 includes one or more of assaying for a mutated protein transcribed from the mutated *NT5C2* gene detected using a method from the group comprising immunostaining, immunoprecipitation, immunoelectrophoresis, Western blotting (Immunoblotting), and nucleotidase enzymatic assays.

In step 411, it is determined whether the biomarker is detected based on the assay performed in step 403. If not, then there is no indication that the subject is likely to resist conventional treatment and step 413 is performed to apply standard nucleoside analog therapy, such as with a combination of 6-MP and 6-TG. Note that step 411 may be performed initially upon diagnosis of ALL, or after treatment has been applied and suspended because of detection of the biomarker, only to be reinstated when the biomarker is no longer detected. This may occur if the alternative treatment performed in step 413, described below, is effective at killing the cell lines with the *NT5C2* mutations that produce the biomarker.

If the biomarker is detected in step 411, then a modified treatment is applied in step 415. For example, a bioactive agent, such as nelarabine, that is not resisted by the *NT5C2* mutations, as described in more detail below, is used in addition to or instead of the conventional nucleoside analog therapy agents 6-MP and 6-TG. In some embodiments, the modified treatment includes the application of ligands that bind with at least the mutated *NT5C2* proteins to keep them from dephosphorylating the metabolic products of the conventional nucleoside analog therapy. In some embodiments, step 415 includes one or more screening methods to identify ligands that bind with high affinity to *NT5C2* protein, which ligands either prevent binding of *NT5C2* protein to a nucleoside analog metabolites, or otherwise prevent the *NT5C2* protein from inactivating the nucleoside analog agents. For example, an anthraquinone derivative (AdiS) inhibitor of NT5C2 is described in Jordheim et al., 2013.

Following either step 413 or step 415, in step 421 it is determined whether it is again time to test for the biomarker. For example, after initial diagnosis and several months of treatment, it is determined to be time to test for the biomarker to see if the patient remains or has become resistant to conventional nucleoside analog therapy. If so, then steps 401 and following are executed again. If not, then in step 422 it is determined whether the process should end, e.g., due to long time remission, cure or patient death. If so, then the process ends. If not, then after a suitable wait, it is determined in step 421 again whether it is time to re-test.

Thus, a teaching is directed to early detection of ALL relapse, by (a) identifying a subject that is at risk of developing relapse ALL or has symptoms of relapse ALL, (b) obtaining a biological sample comprising leukemia cells from the subject; (c) determining if the nucleic acid in the leukemia cells comprises a mutated *NT5C2* gene variant, and (d) if the gene variant is detected, then determining that the subject is at risk of developing relapsed ALL or has relapsed ALL. In a further embodiment, a subject identified above as having relapsed or being at risk of relapsing ALL, is identified as being resistant to conventional nucleoside therapy, for example with 6-mercaptopurine and 6-thioguanine, and such therapy is either discontinued or is not administered. Other methods of treatment are administered instead of or in addition to the conventional therapy.

In another teaching, a subject who has been identified as having one of the hereindefined *NT5C2* mutations is monitored to see if the mutations disappear so that conventional nucleoside treatment can be continued or initiated or returned. This happens in some embodiments, if the treatment the subject is given kills the leukemia cells generated from a clone having the *NT5C2* mutations. In this case, the subject can resume conventional nucleoside treatment.

Other teachings include methods for determining if a subject will respond favorably to conventional nucleoside analog therapy, by: (a) identifying a subject that has ALL, (b) obtaining a biological sample of leukemia cells comprising nucleic acid from the subject; (c) determining if the nucleic acid comprises a mutated *NT5C2* gene variant, and (d) if the mutated gene variant is detected, then determining that the subject will not respond favorably to conventional nucleoside analog therapy.

Furthermore, certain teachings are directed to screening assays for identifying a ligand that binds with high affinity to *NT5C2* protein or to a biologically active fragment or variant thereof, thereby preventing *NT5C2* protein from inactivating a nucleoside analog, comprising (a) screening a ligand library against *NT5C2* protein (or a biologically active fragment or variant thereof) to identify a ligand that binds with high affinity to the *NT5C2* protein, (b) isolating the high affinity ligand from the library; (c) forming a mixture comprising the high affinity ligand, the *NT5C2* protein and the nucleoside analog under conditions that permit the *NT5C2* protein to bind to the nucleoside analog, (d) determining if the *NT5C2* protein binds to the nucleoside analog in the mixture forming an *NT5C2* protein - nucleoside complex, and (e) if the *NT5C2* protein-nucleoside complex is not formed, selecting and identifying the high affinity ligand as one that prevents *NT5C2* protein from inactivating the nucleoside analog (e.g., see Jordheim et al., 2013).

In other similar teachings, screening assays identify ligands that bind with high affinity to *NT5C2* protein thereby preventing the *NT5C2* protein from inactivating a nucleoside analog. In this embodiment, the ligand does not necessarily block binding of the *NT5C2* protein to the nucleoside, but does block inactivation of the nucleoside. In some embodiments, the *NT5C2* protein is a mutated protein encoded by one of the herein-described *NT5C2* gene variants.

Certain screening assays include determining if the binding of the high affinity ligand to the *NT5C2* protein reduces the nucleotidase activity of the hyperactive mutant *NT5C2* proteins.

Libraries for use in embodiments of the invention include a constrained ligand library or a combination library having constrained and unconstrained ligands. The library of compounds comprises small molecules, peptides or antibodies. The ligands can be bound to a support such as is a bead, a chip, a filter, a dipstick, a membrane, a polymer matrix or a well. Alternatively, the *NT5C2* protein or mutant thereof can be so bound while the ligand is in solution.

If it is determined that the subject will not respond favorably to conventional nucleoside analog therapy, the subject can be treated by administering a therapeutically effective amount of a nucleoside analog if it is administered in combination with an amount of a high affinity ligand identified in the above-described screening that prevents inactivation of the nucleoside analog by the mutated *NT5C2* protein in the subject.

If it is determined that the subject will not respond favorably to conventional nucleoside analog therapy, the subject can be treated by administering a therapeutically effective amount of a nucleoside analog if it is administered in combination with a nucleoside analog, such as nelarabine, that is not affected by the *NT5C2* mutated protein in the subject.

### Summary of Experimental Results and Embodiments

Analysis of 5 pediatric ALL patients, all of whom had relapsed at least once, showed there was a mean mutation load of 13 somatic mutations per sample. Whole exome sequencing of matched diagnosis, remission and relapse DNA samples from these 5 human pediatric ALL patients with T-cell immunophenotype identified a mean mutation load of 13 somatic mutations per sample (range 5-17). Out of 60 somatic mutations identified in total, 17 mutations were present at diagnosis and at relapse, 24 genes were selectively mutated in relapsed ALL samples and 19 mutations were present only at diagnosis. Moreover, 4 of the 5 relapsed cases analyzed showed the presence of at least one somatic mutation present also at diagnosis, in addition to secondary mutations specifically acquired at the time of relapse. In addition, 4 out of the 5 cases showed loss of at least one mutation marker present at diagnosis during disease progression leading to relapse. Single nucleotide polymorphism analysis of exome sequencing results ruled out that loss of these markers was due to loss of heterozygosity at relapse This result is consistent with previous studies based on copy number alteration analyses (Bunz, 1999; McCubrey, 2007; Jordheim, 2003) and supports that relapsed ALLs can originate as derivates of ancestral subclones related to, but distinct from the main leukemic population present at diagnosis.

New genes not previously implicated in the pathogenesis of T-ALL were identified. Three new genes were identified at the time of relapse encoding proteins involved in positive regulation of TP53 signaling, including TP53 itself (*TP53 R213Q*), BANP (*BANP H391Y*) and RPL11 (*RPL11 R18P*). Although *TP53 R213* has been described in cancer before, it was not associated with T-ALL. Two new additional somatic RPL11 mutant alleles were identified; one present both at diagnosis and relapse (*RPL11 X178Q*); and the other one (*RPL11 G30fs*) specifically mutated at relapse.

Two diagnostic and relapse sample pairs harboring a prototypical NRAS G12S activating allele and a third patient with a heterozygous activating NRAS G12R mutation present at diagnosis, which showed loss of heterozygosity at the time of relapse were identified.

One remarkable finding in the exome sequence analysis was the presence of a relapse-associated heterozygous mutation in the cytosolic 5'-nucleotidase II gene (*NT5C2 K359Q*), which encodes an enzyme responsible for the inactivation of nucleoside analog antitumor drugs (Jordheim, 2003). Extended mutation analysis of *NT5C2* in diagnosis and relapse ALL patients identified 23 additional patients harboring *NT5C2* mutations (*NT5C2* gene variants) acquired at the time of relapse, bringing the frequency of relapse-associated *NT5C2* mutations to 16% (23/138) at the time. Strikingly, 15 of the 23 relapse associated *NT5C2* mutations in independent relapsed patients harbored the same *NT5C2 R367Q* mutation. The *R367Q* mutation is the most frequent and the most important mutation which suggests it should be among the variants screened to determine relapse or risk of relapse or nucleoside therapy resistance, if the number of *NT5C2* gene variants tested is limited.

Continued data collection identified the 138 relapsed ALL patients mentioned above. Twenty-four (24) of the relapsed patient samples in the study are listed in Table 2. All listed show *NT5C2* mutations.

**Table 2. Example NT5C2 protein mutations in ALL**

| **Sample ID** | **Diagnosis** | **Exon** | **Predicted amino acid change** | **Ref. Seq NM_012229** |
|---|---|---|---|---|
| Relapse T-ALL 4 | T-ALL | 13 | R367Q | 1100G>A |
| Relapse T-AL111 | T-ALL | 13 | K359Q | 1076A>C |
| Relapse T-ALL 17 | T-ALL | 13 | R367Q | 1100G>A |
| Relapse T-ALL 17 | T-ALL | 9 | R238L | 713G>T |
| Relapse T-ALL 22 | T-ALL | 9 | R238W | 712C>T |
| Relapse T-ALL 29 | T-ALL | 11 | R291W | 871C>T |
| Relapse T-ALL 35 | T-ALL | 13 | R367Q | 1100G>A |
| Relapse T-ALL 37 | T-ALL | 15 | D407A | 1219A>C |
| Relapse T-ALL 30 | T-ALL | 17 | Q523* | 1567C>T |
| Relapse pre-B ALL 16 | pre-B ALL | 13 | R367Q | 1100G>A |
| Relapse T-ALL B11 | T-ALL | 13 | R367Q | 1100G>A |
| Relapse T-ALL B15 | T-ALL | 13 | R367Q | 1100G>A |
| Relapse T-ALL B30 | T-ALL | 13 | R367Q | 1100G>A |
| Relapse T-ALL B37 | T-ALL | 13 | R367Q | 1100G>A |
| Relapse T-ALL B39 | T-ALL | 13 | R367Q | 1100G>A |
| Relapse T-ALL B44 | T-ALL | 13 | R367Q | 1100G>A |
| Relapse T-ALL B48 | T-ALL | 13 | R367Q | 1100G>A |
| Relapse T-ALL B52 | T-ALL | 13 | R367Q | 1100G>A |
| Relapse T-ALL B53 | T-ALL | 13 | R367Q | 1100G>A |
| Relapse T-ALL B9 | T-ALL | 9 | R238W | 712C>T |
| Relapse T-ALL B29 | T-ALL | 9 | R238W | 712C>T |
| Relapse T-ALL B64 | T-ALL | 9 | R238W | 712C>T |
| Relapse T-ALL B9 | T-ALL | 13 | L375F | 1123C>T |
| Relapse T-ALL B64 | T-ALL | 13 | L375F | 1123C>T |

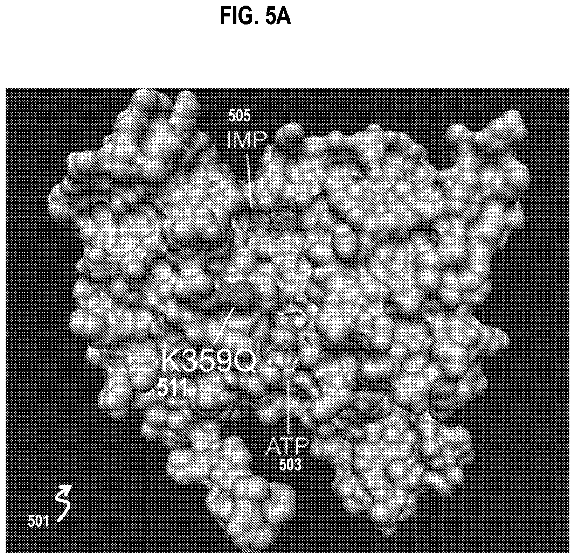

FIG. 5A is a molecular surface representation diagram of the *NT5C2 WT* protein 501 structure with a location 511 of the *K359Q* mutation and binding sites 503 and 505 for ATP and inosine monophosphate (IMP), respectively, according to some embodiments. The substrate inosine monophosphate (IMP) 505 is depicted along with the ATP allosteric activator 503. The *K359Q* mutation is located at site 511 where *K359Q* can affect both the IMP binding and the ATP binding.

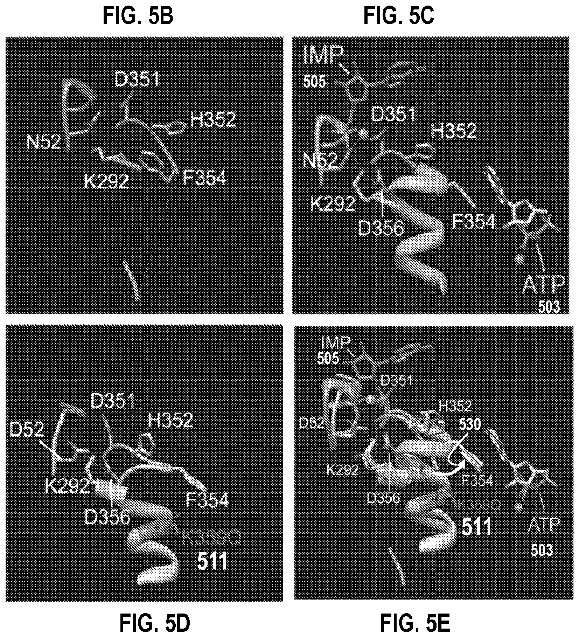

FIG. 5B through FIG. 5E are diagrams showing the relative locations on the *NT5C2* protein of *K359Q* and other amino acids and IMP and ATP binding sites; according to various embodiments. FIG. 5B indicates relative locations of amino acids (by letter-number to indicate wild type amino acid and location) N52, K292, D351, H352 and F354. This depicts a structure representation of the *NT5C2* catalytic center and allosteric regulatory site devoid of substrate or ligands (PDB 2XCX). FIG. 5C indicates relative locations of the same amino acids plus amino acid D356 and the binding of IMP and ATP at sites 505 and 503, respectively. This depicts a structure representation of the *NT5C2* catalytic center and allosteric regulatory site bound to IMP and ATP, respectively (PDB 2XCW). FIG. 5D indicates relative locations of the same amino acids as in FIG. 5C plus mutation *K359Q* 511. This depicts a structure representation of the *NT5C2 K359Q* mutant model corresponding to the catalytic center and allosteric regulatory sites. The group that solved the structure of the NT5C2 protein introduced some artificial changes to improve protein stability, solubility and crystallization. These include the truncation of the C-terminus part of the protein (therefore not included in the structure) and the mutation of amino acid 52 from N to D. This latter structure was used to model the effect of the K359Q mutation. FIG. 5E indicates relative locations of the same amino acids and mutations as in FIG. 5D plus the binding of IMP and ATP at sites 505 and 503, respectively. This depicts an overlay of the structures shown above. The white arrow 530 indicates the repositioning of F354 from the inactive *NT5C2* configuration to the active-ATP-bound *NT5C2* and *NT5C2 K359Q*-structures. Ions of Mg²⁺ are depicted as light spheres.

Comparison between the distances of electrostatic interactions in the wild type and mutant *NT5C2* structures suggest a retained active site pocket that is capable of binding nucleoside monophosphate substrates and nucleoside analogs (FIG. 5A). Moreover, these models predict negligible structural changes to critical sites D52 and D54, responsible for the nucleophilic attack on the phosphate moiety of the substrate, and the subsequent donation of a proton to the departing nucleoside product (Wallden, 2011). To further assess the functional significance of leukemia relapse associated *NT5C2* mutations, the effects of these alterations on the allosteric regulation of *NT5C2* was examined. *NT5C2* is positively regulated by the binding of activating effectors (ATP, dATP, Ap₄A and 2,3-BPG) to an allosteric pocket proximal to the active site. Occupancy of this allosteric site results in increased ordering of an alpha helix formed by residues G355-E364 (Helix A) and an activating conformational change that displaces F354 from the catalytic center and moves D356 into the active site of the protein. Strikingly, structural analysis using the Site Directed Mutator (SDM) statistical potential energy function algorithm (Worth, 2011) showed that the *NT5C2 K359Q* mutation in the Helix A region of the protein mimics the effect of these positive allosteric regulators. Thus, *NT5C2 K359Q* increases the Helix A stability and reduces its solvent accessibility, which results in an active configuration with displacement of F354 out of the active site and positioning D356 into the catalytic center of the enzyme (FIG. 5B through FIG. 5E).

Given the described role of *NT5C2* in the metabolism and inactivation of nucleoside analog drugs (Brouwer, 2005; Hunsucker, 2005; Galmarini, 2003a); the recurrent finding of certain alleles including the *NT5C2 R367Q, NT5C2 R238W* and *NT5C2 L375F* alleles; and the reported association of increased levels of nucleotidase activity with thiopurine-resistance and worse clinical outcome (Pieters, 1992); it was hypothesized that relapse-associated *NT5C2* mutations may represent gain of function alleles with increased enzymatic activity. Structural analysis using the Site Directed Mutator (SDM) statistical potential energy function algorithm (Hof, 2011) showed that the *NT5C2 K359Q* mutation in the Helix A region of the protein mimics the effect of these positive allosteric regulators. Thus, the mutant *NT5C2 K359Q* protein has increased helix stability and reduced solvent accessibility.

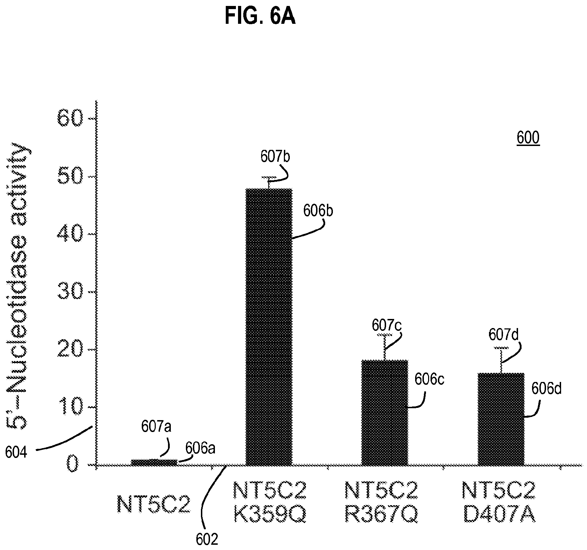

In addition, and despite the absence of clear structural cues suggesting a role of other mutations in *NT5C2* activation, analysis of the *NT5C2 R367Q* and *NT5C2 D407A* mutant proteins in this assay revealed an 18-fold and a 16-fold increase in their 5'-IMP nucleotidase activity compared with wild type *NT5C2,* respectively. FIG. 6A is a bar graph 600 that illustrates example increased activity in three *NT5C2* mutant proteins compared to wild type, according to an embodiment. 5'-Nucleotidase activity levels are determined as described below in the materials and methods section. The horizontal axis 602 indicates the protein or mutation; the vertical axis 604 indicates relative level of 5'-nucleotidase activity in arbitrary units normalized to wild type activity. Mean activity levels and standard deviations observed are indicated by bar height 606a and standard deviation 607a for *NT5C2 WT* (control), bar height 606b and standard deviation 607b for *NT5C2 K359Q,* bar height 606c and standard deviation 607c for *NT5C2 R367Q,* and bar height 606d and standard deviation 607d for *NT5C2 D407A.* 5'-Nucleotidase activity levels of recombinant mutant proteins are shown to be much higher than wild type *NT5C2* (control). The bar height 606a of wild type equals 1, by definition. The most activity of the mutations depicted is associated with mutation *NT5C2 K359Q,* which is about 47 times the activity of the wild type.

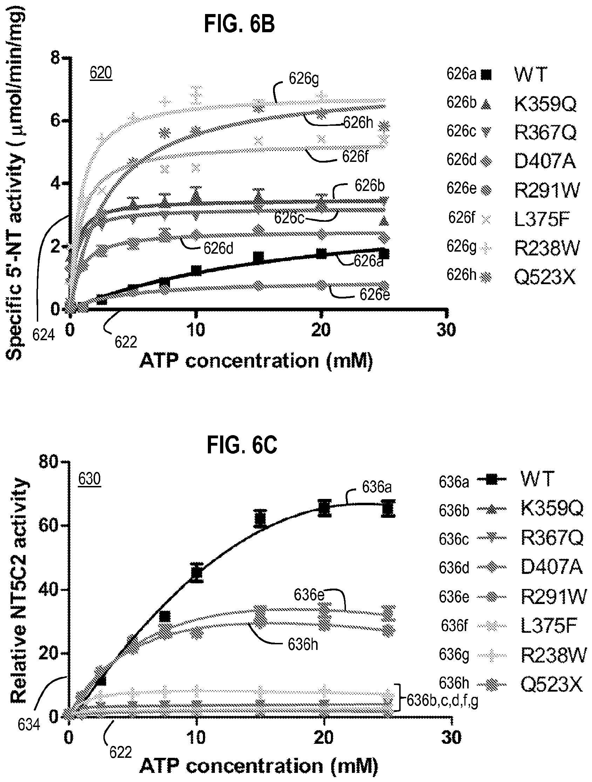

FIG. 6B through FIG. 6D are graphs that illustrate example increased activity and less dependence on ATP concentration for at least six of seven *NT5C2* mutant proteins compared to wild type, according to various embodiments. FIG. 6B is graph 620 of specific 5'-nucleotidase (NT) activity as a function of ATP concentration. The horizontal axis 622 indicates ATP concentration in milliMolar (mM, 1 mM = 10⁻³ Molar); and, the vertical axis 624 indicates specific 5'-NT activity in microMoles per minute (min) per milligram (1 microMole, mM, = 10⁻⁶ moles; 1 milligram, mg, = 10⁻³ grams). Trace 626a indicates the dependence of wild type activity on ATP, and shows a steady increase in activity with ATP concentration. Trace 626b for mutation *K359Q,* trace 626c for *R367Q,* trace 626d for *D407A,* trace 626e for *R291W,* trace 626f for *L375F* and trace 626g for *R238W* show rapid rise to near maximum activity, and then little dependence on ATP concentration. Only trace 626h for mutation *Q523X,* among the mutations, shows a gradual rise to maximum activity. All mutations except *R291W* (trace 626e) show higher specific activity than the wild type (trace 626a). *K359Q* shows a small standard deviation relative to the activity level and a good fit between data points and trace 626b.

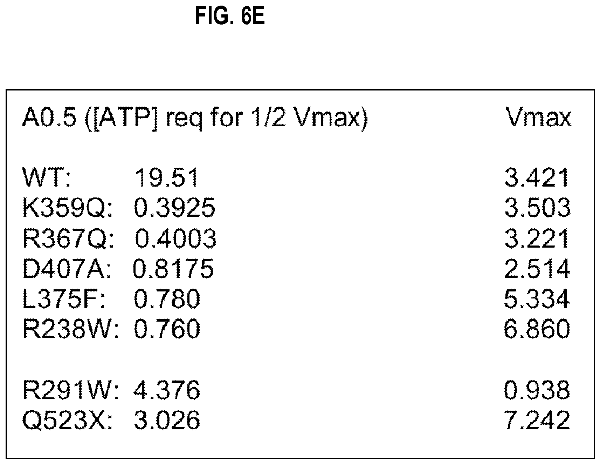

FIG. 6C is graph 630 of 5'-nucleotidase (NT) activity as a function of ATP concentration relative to activity in the absence of ATP (0 mM ATP). The horizontal axis 622 is as in FIG. 6B; and, the vertical axis 634 indicates 5'-NT activity relative to 0 mM ATP and is dimensionless. Trace 636a indicates the dependence of wild type relative activity on ATP, and shows a steady increase in activity with ATP concentration by a factor over 60 at ATP concentrations over 20 mM. The only mutations showing relative active increases over a factor of 20 are trace 626e for *R291W* and trace 636h for mutation *Q523X.* The other traces, including trace 626b for mutation *K359Q,* trace 626c for *R367Q,* trace 626d for *D407A,* trace 626f for *L375F* and trace 626g for *R238W* show little change between reference activity and maximum activity. This point is reiterated in FIG. 6E. FIG. 6E is a table that illustrates less dependence of activity on ATP concentration for seven *NT5C2* mutant proteins compared to wild type, according to various embodiments. Vmax is maximum specific activity as plotted in FIG. 6B. A0.5 is the ATP concentration to achieve an activity equal to half Vmax. The wild type needs 19.51 mM of ATP to achieve half Vmax. Two mutations *R291W* and *Q523X* require a small fraction of this at about 4 mM ATP and 3 mM ATP, respectively, to achieve half Vmax. The remaining mutations achieve half of maximum activity at less than 1 mM ATP. Thus activity is nearly independent of ATP concentration.

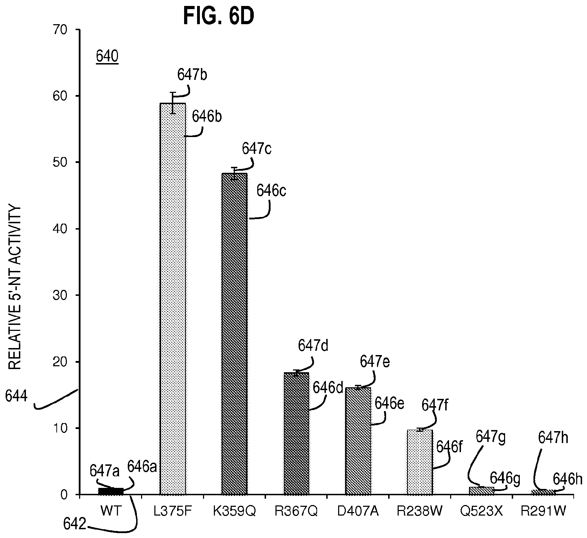

FIG. 6D is a graph 640 that shows 5'NT activity of seven mutations relative to the 5'NT activity of the wild type protein *NT5C2 WT.* The horizontal axis 642 indicates the protein or mutation; the vertical axis 644 indicates relative level of 5'-nucleotidase activity in arbitrary units normalized to wild type activity. Mean activity levels and standard deviations observed are indicated by bar height 646a and standard deviation 647a for *NT5C2 WT* (control), bar height 646b and standard deviation 647b for *NT5C2 L375Q,* bar height 646c and standard deviation 647c for *NT5C2 K359Q,* bar height 646d and standard deviation 647d for *NT5C2 R367Q,* bar height 646e and standard deviation 647e for *NT5C2 D407A,* bar height 646f and standard deviation 647f for *NT5C2 R238W,* bar height 646g and standard deviation 647g for *NT5C2 Q523X,* and bar height 646h and standard deviation 647h for *NT5C2 R291W.* 5'-Nucleotidase activity levels of recombinant mutant proteins are shown to be much higher than wild type *NT5C2* (control). The bar height 606a of wild type equals 1, by definition. The activity associated with mutation *NT5C2 K359Q* is about 47 times the activity of the wild type, as described above. The most activity is associated with mutation *L375F,* which is almost 60 times the activity of the wild type. Six mutations have activities that are significantly higher than the activity of the wild type. Only mutation *NT5C2 R291W* has activity that is not significantly different from the wild type activity. At least the six *NT5C2* mutations are thus expected to be effective at neutralizing the effectiveness of the 6-MP and 6-TG nucleoside analogs of the conventional treatment.

To formally test the role of *NT5C2* mutations in chemotherapy resistance, the effects of wild type and relapse-associated mutant *NT5C2* expression were analyzed in the response of CCRF-CEM T-ALL cells to 6-mercaptopurine (6-MP) and 6-thyogunanine (6-TG). Expression of *NT5C2* mutations in ALL cells induces resistance to chemotherapy with 6-MP and 6-TG. Viability assays were performed in CCRF-CEM and CUTLL1 T-ALL cells expressing wild type *NT5C2,* relapse-associated mutant *NT5C2* alleles, or a red fluorescent protein (RFP) as control, treated with increased concentrations of the nucleoside analog chemotherapeutic drugs.

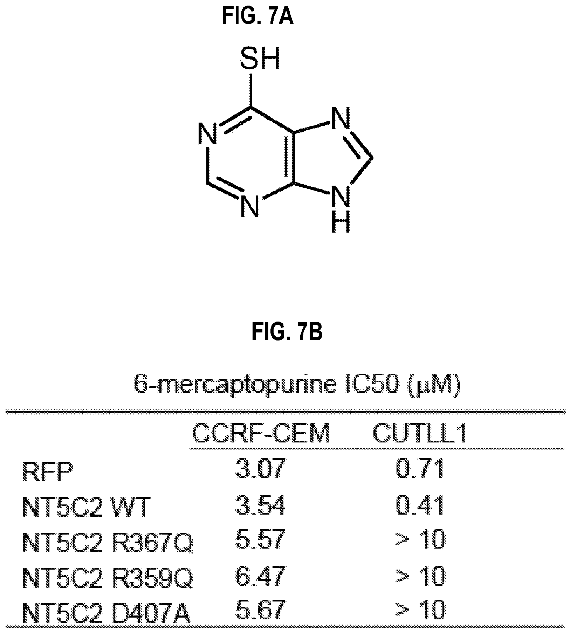

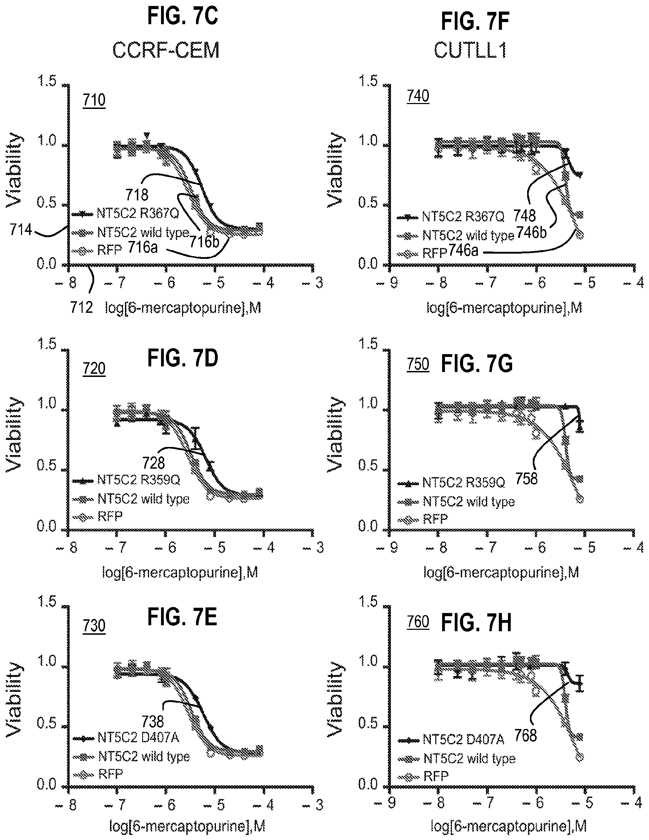

FIG. 7A is a diagram of the nucleoside analog 6-mercaptopurine (6-MP). FIG. 7B is a table that illustrates higher resistance of cells expressing three *NT5C2* mutant proteins in the presence of 6-MP in two cell lines, according to various embodiments. IC50 is resistance as indicated by concentration in microMolar, µM, of 6-MP to diminish viability to 50%. The cell lines are CCRF-CEM and CUTLL1 T-ALL. The proteins expressed in the different experiments are a red fluorescent protein (RFP) control, a wild type *NT5C2 WT,* relapse-associated mutant *NT5C2* alleles *NT5C2 R367Q, NT5C2 R3*59Q, and *NT5C2 D407A.* When subjected to 6-MP, the concentration needed to reduce viability to 50% (IC50) is greater for these mutations than the control (RFP) or wild type (*NT5C2 WT*), which are similar to each other. The increase is about double in the CCRF-CEM cell line and greater than ten times as much in the CUTLL1 cell line, indicating substantial resistance to the conventional nucleoside analog therapy.

FIG. 7C through FIG. 7H are graphs 710, 720, 730, 740, 750, 760, respectively, that illustrate example higher viability of cells expressing three *NT5C2* mutant proteins, respectively, in the presence of various doses of 6-MP in two cell lines, respectively, according to various embodiments. Each graph has the same horizontal axis 712 that indicates a log of the concentration of 6-MP in Molar; and, the same vertical axis 714 that indicates cell viability, in terms of cell population count (e.g., at 48 hours) relative to count in control cells treated only with the vehicle solution, and is dimensionless. Graphs 710, 720, 730 show viability of the cell line CCRF-CEM expressing one of three mutant proteins, *NT5C2 R367Q, NT5C2 R359Q,* and *NT5C2 D407A,* respectively, relative to RFP (open circles) 716a and *NT5C2 WT* (closed squares) 716b plotted on each graph, which are roughly the same. In graph 710 the viability of CCRF-CEM cells expressing mutation *R367C* is given by trace 718 (down solid triangles) is greater than control and wild cells for log concentrations between about -6 and -4.5. The log concentration at which viability falls to 50% is about -5 for the mutation and about -5.2 for the control or wild proteins. That is, a higher concentration of 6-MP is needed to reduce viability of CCRF-CEM cells expressing the mutation than needed to reduce by the same amount the viability of cells expressing the control or wild type proteins. Similar results are shown for the other mutations, *R359Q* trace 728 (up solid triangles) in graph 720 and *D407A* trace 738 (solid circles) in graph 730.

Graphs 740, 750, 760 show viability of the cell line CUTLL1 expressing one of three mutant proteins, *NT5C2 R367Q, NT5C2 R359Q,* and *NT5C2 D407A,* respectively, relative to RFP (open circles) trace 746a and *NT5C2 WT* (closed squares) trace 746b plotted on each graph. CUTLL1 cells expressing the *NT5C2* wild type show greater viability than those expressing the RFP control. In graph 740 the viability of CUTLL1 cells expressing mutation *R367C* is given by trace 748 (down solid triangles), and is greater than control and wild cells for some log concentrations about -5. The log concentration at which viability falls to 50% is not included in the data for the mutation, and is about -5.2 for the control or wild proteins. That is, a higher concentration of 6-MP is needed to reduce viability of CUTLL1 cells expressing the mutation than needed to reduce by the same amount the viability of cells expressing the control or wild type proteins. Similar results are shown for the other mutations, *R359Q* trace 758 (up solid triangles) in graph 750 and *D407A* trace 768 (solid circles) in graph 760.

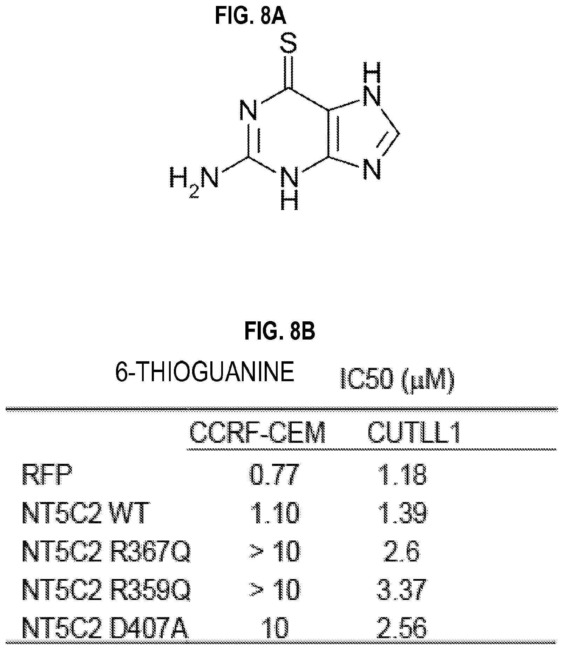

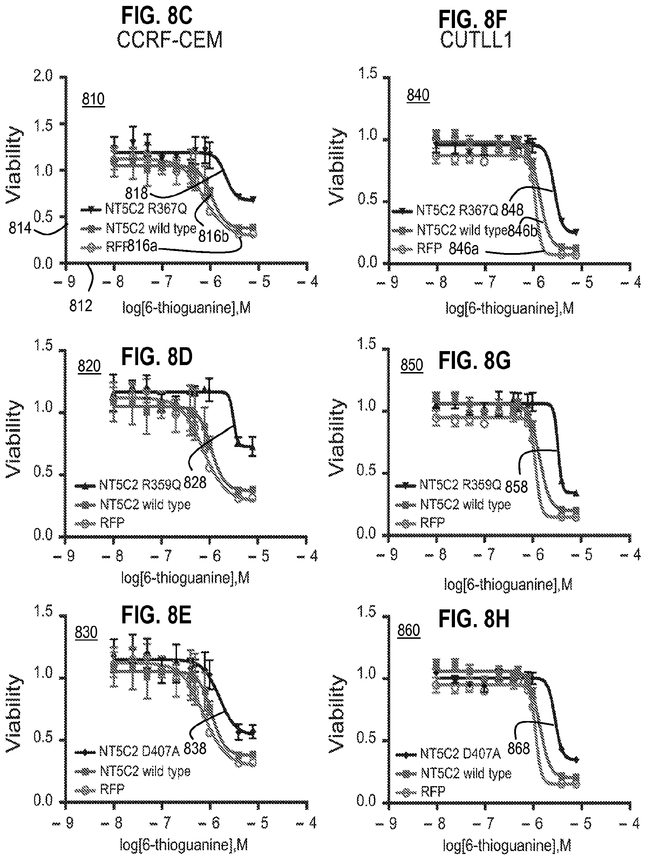

FIG. 8A is a diagram of the nucleoside analog 6-thioguanine (6-TG). FIG. 8B is a table that illustrates higher resistance of cells expressing three mutant *NT5C2* proteins in the presence of 6-TG in two cell lines expressed as IC50 concentrations, according to various embodiments. The cell lines and proteins expressed are the same as used in FIG. 7B for 6-MP. Here the mutations again provide greater viability than the control RFP or wild type *NT5C2* proteins, with the more dramatic increase in viability appearing in the CCRF-CEM cell line. When subjected to 6-TG, the concentration needed to reduce viability to 50% is greater for these mutations than the control (RFP) or wild type (*NT5C2 WT*), which are similar to each other. The increase is about double in the CUTLL1 cell line and greater than ten times as much in the CCRF-CEM cell line, indicating substantial resistance to the conventional nucleoside analog therapy

FIG. 8C through FIG. 8H are graphs 810, 820, 830, 840, 850, 860, respectively, that illustrate example higher viability of cells expressing three *NT5C2* mutant proteins, respectively, in the presence of various doses of 6-TG in two cell lines, respectively, according to various embodiments. Each graph has the same horizontal axis 812 that indicates a log of the concentration of 6-TG in Molar; and, the same vertical axis 814 that indicates cell viability, in terms of cell population count relative to count in vehicle only treated cells, and is dimensionless. Graphs 810, 820, 830 show viability of the cell line CCRF-CEM expressing one of three mutant proteins, *NT5C2 R367Q, NT5C2 R359Q,* and *NT5C2 D407A,* respectively, relative to RFP (open circles) 816a and *NT5C2 WT* (closed squares) 786b plotted on each graph, which are roughly the same. In graph 810 the viability of CCRF-CEM cells expressing mutation *R367C* is given by trace 818 (down solid triangles) is greater than control and wild cells for log concentrations between about -6.5 and -5. The log concentration at which viability falls to 50% is outside the collected data for the mutation, and is about -6 for the control or wild proteins. That is, a higher concentration of 6-TG is needed to reduce viability of CCRF-CEM cells expressing the mutation than needed to reduce by the same amount the viability of cells expressing the control or wild type proteins. Similar results are shown for the other mutations, *R359Q* trace 828 (up solid triangles) in graph 820 and *D407A* trace 838 (solid circles) in graph 830.

Graphs 840, 850, 860 show viability of the cell line CUTLL1 expressing one of three mutant proteins, *NT5C2 R367Q, NT5C2 R359Q,* and *NT5C2 D407A,* respectively, relative to RFP (open circles) trace 846a and *NT5C2 WT* (closed squares) trace 846b plotted on each graph. CUTLL1 cells expressing the *NT5C2* wild type show greater viability than those expressing the RFP control. In graph 840 the viability of CUTLL1 cells expressing mutation *R367C* is given by trace 848 (down solid triangles) and is greater than control and wild cells for some log concentrations below about -5. The log concentration at which viability falls to 50% is about -5.5 for the mutation and about -6 for the control or wild proteins. That is, a higher concentration of 6-TG is needed to reduce viability of CUTLL1 cells expressing the mutation than needed to reduce by the same amount the viability of cells expressing the control or wild type proteins. Similar results are shown for the other mutations, *R359Q* trace 858 (up solid triangles) in graph 850 and *D407A* trace 868 (solid circles) in graph 860.

Cell viability analysis in the presence of increased drug concentrations demonstrated increased resistance to 6-MP and 6-TG therapy in cells expressing *NT5C2 K359Q, NT5C2 R367Q* and *NT5C2 D407A* compared with empty vector and wild type *NT5C2* controls. Similar results were obtained in the CUTLL1 T-ALL cell line.

Mutation analysis performed of an extended panel of 103 relapse T-ALL and 35 relapse B-precursor ALL samples identified 22 additional T-ALL patients and one B-precursor ALL patient harboring *NT5C2* mutations in first relapse. Strikingly, 13 of these samples harbored the same *NT5C2 R367Q* mutation, 4 cases showed a recurrent *NT5C2* R238W mutation and 2 samples harbored a *L375F* single amino acid substitution. In each of the 9 cases for which original diagnostic DNA was available for analysis, *NT5C2* mutations showed to be specifically acquired at the time of relapse. No *NT5C2* mutations were identified in 23 T-ALL and 27 B-precursor ALL additional diagnostic samples, further supporting the specific association of *NT5C2* mutations with relapsed disease. Analysis of clinical and molecular features associated with *NT5C2* mutant relapsed T-ALLs treated in Berlin Frankfurt Münster (BFM) group based clinical trials (ALL-BFM 95, ALL-BFM 2000, COALL 06-97, NHL-BFM 95 and Euro-LB 02) showed an association of *NT5C2* mutations with early disease recurrence (very early or early relapse *vs.* late relapse, *P*<0.05) and relapse under treatment (*P*=0.002).

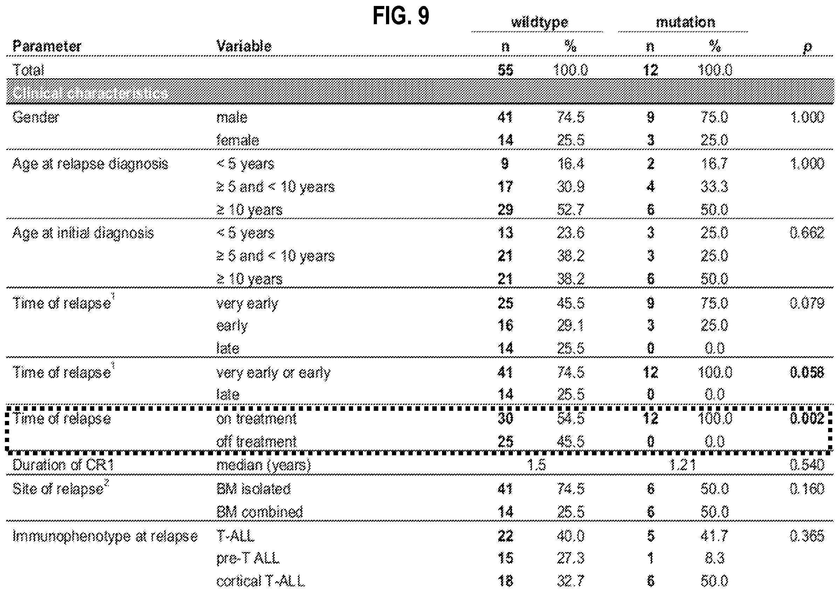

FIG. 9 is a table that illustrates an example distribution of mutant *NT5C2* proteins in patients who relapse, according to an embodiment. 100% of patients who relapse while on conventional nucleoside analog treatment express a mutant *NT5C2* protein. In each of 9 cases for which original diagnostic DNA was available for analysis, *NT5C2* mutations showed to be specifically acquired at the time of relapse. No *NT5C2* mutations were identified in 23 T-ALL and 27 B-precursor ALL additional diagnostic samples, further supporting the specific association of *NT5C2* mutations with relapsed disease. Analysis of clinical and molecular features associated with *NT5C2* mutant relapsed T-ALLs treated in Berlin Frankfurt Münster (BFM) group based clinical trials (ALL-BFM 95, ALL-BFM 2000, COALL 06-97, NHL-BFM 95 and Euro-LB 02) showed an association of *NT5C2* mutations with early disease recurrence (very early or early relapse vs. late relapse, P <0.05) and relapse under treatment (P =0.002). For FIG. 9, the following terms are defined. Abbreviations: BM, bone marrow; CR2, second complete remission; CCR, complete continuous remission; MRD, minimal residual disease in bone marrow aspiration after induction phase (week 5); HSCT, hematopoietic stem cell transplantation. Time of relapse: very early, <18 months after initial diagnosis of ALL; early, ≥18 months after initial diagnosis of ALL; late, ≥6 months after completion of primary treatment. Site of relapse: isolated BM, no evidence of extramedullary disease; combined BM, more than 5% leukemic cells in the BM combined with CNS, testis, or other extramedullary disease. Cytologic response: early, remission after first induction course; normal, remission after second induction course; late, remission after 1st R2 block/day 15 protocol II-IDA; non-response, no remission after 1st R1 block/day 29 protocol II-IDA. Cytologic remission was defined as less than 5% leukemic blasts in regenerating bone marrow, no peripheral blasts cells and no extramedullary involvement.

The effects of relapse-associated *NT5C2* mutations in the response to nelarabine were tested. Nelarabine is a mixture of an AraG precursor highly active in relapsed T-ALL-and AraG (Sanford, 2008; Berg, 2005; Gokbuget, 2011; DeAngelo, 2007). Strikingly, both nelarabine and AraG showed to be equally active in cells expressing relapse-associated *NT5C2* mutations compared to controls. This means that treatment with nelarabine can be continued in relapse or at risk subjects having the herein-described *NT5C2* gene variants.

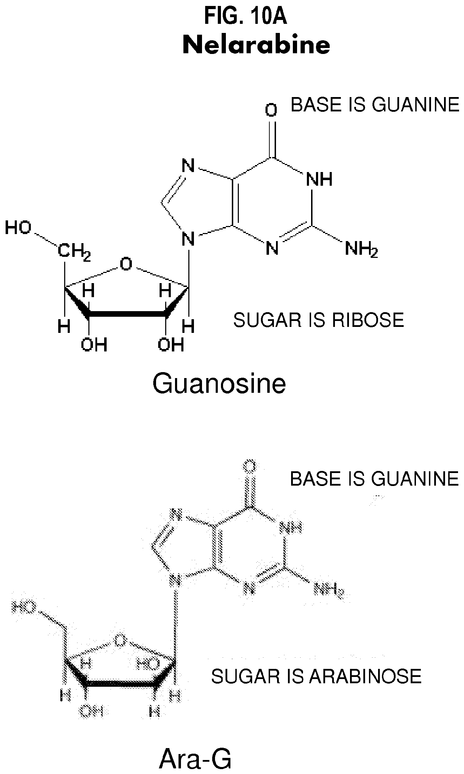

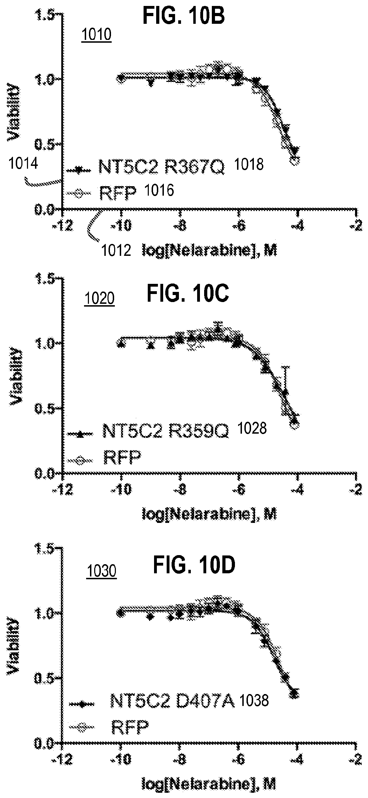

FIG. 10A is a diagram of the nucleoside analog nelarabine. FIG. 10B through FIG. 10D are graphs 1010, 1020 and 1030 that illustrate example similar viability of cells expressing control protein RFP and three *NT5C2* mutant proteins, respectively, in the presence of various doses of nelarabine, according to various embodiments. Each graph has the same horizontal axis 1012 that indicates a log of the concentration of nelarabine in Molar, and the same vertical axis 1014 that indicates cell viability, in terms of cell population count relative to count in vehicle only treated controls, and is dimensionless. Graphs 1010, 1020, 1030 show viability of a CCRF-CEM cell line expressing one of three mutant proteins, *NT5C2 R367Q, NT5C2 R359Q,* and *NT5C2 D407A,* respectively, relative to RFP (open circles) 1016 plotted on each graph. In graph 1010 the viability of cells expressing mutation *R367C* is given by trace 1018 (down solid triangles) is not significantly different from the control protein. The log concentration at which viability falls to 50% is about -4 for the control and mutant proteins.. Similar results are shown for the other mutations, *R359Q* trace 1028 (up solid triangles) in graph 1020 and *D407A* trace 1038 (solid circles) in graph 1030. That is, the mutations of *NT5C2* do not confer resistance to nelarabine

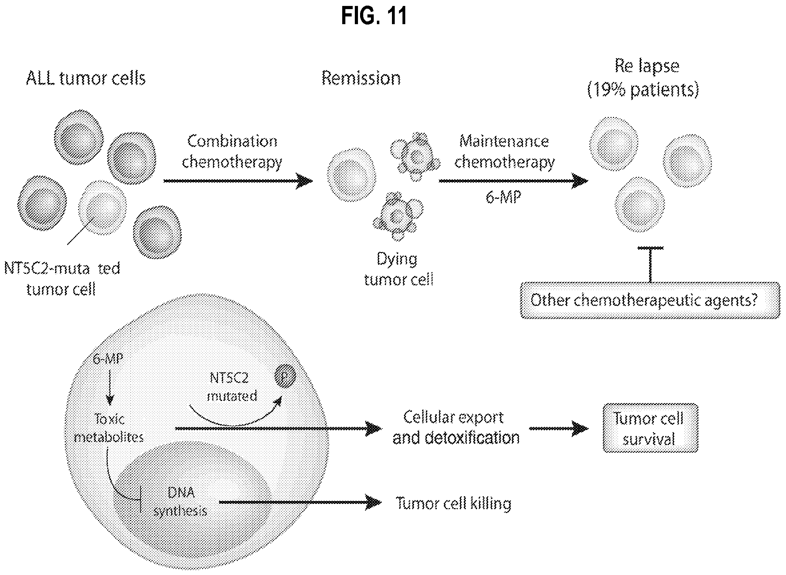

For purposes of illustration an example model of tumor development is presented here; however, embodiments of the invention are not limited by the accuracy or completeness of this model. FIG. 11 is a diagram that illustrates an example model for ALL relapse during treatment in the presence of *NT5C2* mutant proteins, according to an embodiment. Initially there is a population of ALL tumor cells (not shown). At some point one or more cells develop a mutation of the *NT5C2* protein that confers resistance to conventional combination nucleoside analog therapy (e.g., 6-MP and 6-TG). As a result of this therapy the non-mutated tumor cells die, while the *NT5C2* mutated tumor cells resist the conventional treatment and survive. During maintenance chemotherapy with 6-MP alone the *NT5C2* mutated cells multiply which leads to relapse in about 19% of patients. Effective treatment of the relapsed patient advantageously involves other chemotherapeutic agents.

For purposes of illustration, a proposed mechanism for the resistance to 6-MP is illustrated in the enlarged cell at the bottom of FIG. 11. However, embodiments of the invention are not limited to the accuracy or completeness of this mechanism. When 6-MP enters the cell it generates toxic metabolites which adversely affect DNA synthesis and lead to killing of the tumor cell. In cells expressing mutated *NT5C2* proteins, the toxic metabolites tend to undergo detoxification and cellular export, leading to tumor cell survival.

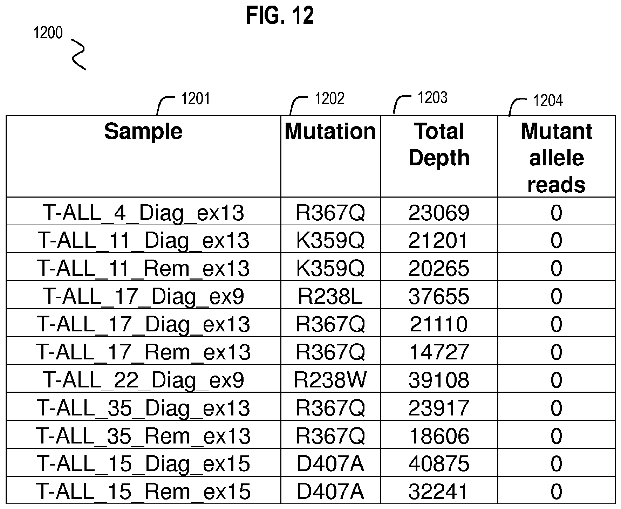

FIG. 12 is a table 1200 that illustrates example data finding no *NT5C2* mutations in ALL patients who are originally diagnosed with the disease or in remission, according to an embodiment. Eleven samples are included in the table, all of which were taken at time of original diagnosis or remission. The samples are identified in the first column 1201. The mutation searched for by sequencing the DNA in the vicinity of the corresponding SNP in the corresponding exon is listed in second column 1202. The number of times the vicinity was sequenced, which is the total depth of the sequencing, is given in the third column 1203. The number of mutant allele reads among those sequences is given in the fourth column 1204. Despite total depths between about 1500 and about 4000, no mutant allele reads were obtained, indicating that the mutations are extremely rare and essentially absent at original diagnosis and remission. Thus finding such alleles in 12 of 55 relapsed patients listed in FIG. 9 is extremely significant. In fact, such alleles are found in 100% of patients who relapse early or during treatment.

Prolonged maintenance treatment with 6-mercaptopurine is advantageous to obtain durable remissions in the treatment of ALL (Clappier, 2011; Thompson, 2007). Indeed, low-adherence to 6-mercaptopurine treatment, defined as less than 95% compliance, results in increased relapse rates and may account for as much as 59% of all ALL relapses (Bhatia, 2012). In this context, the results described herein highlight the prominent role of relapse-specific mutations in *NT5C2* as a mechanism of resistance to 6-MP and a genetic driver of relapse in ALL. In addition, and most notably, the lack of nelarabine cross-resistance in cells expressing activating *NT5C2* alleles analyzed here, suggests that these mutations may not impair the effectiveness of nelarabine-based salvage therapies in relapse T-ALL.

Overall, these results demonstrate that chemotherapy-driven clonal selection is a common driver mechanism of leukemia relapse and highlight the prominent role of relapse-specific mutations in *NT5C2* in disease progression and chemotherapy resistance in ALL. This finding supports the embodiments directed to methods of diagnosis and screening assays to develop specific *NT5C2* inhibitors or chemotherapeutic alternatives to be administered therapeutically to avoid nucleoside-analog chemotherapy resistance in the treatment of relapsed ALL.

### Materials and Methods

### Patient samples.

DNAs from leukemic ALL blasts at diagnosis and relapse and matched remission lymphocytes were provided by the Hemato-Oncology Laboratory, Department of Pediatrics, University of Padua, Padua, Italy. All samples were collected in clinical trials with informed consent and under the supervision of local IRB committees. Consent was obtained from all patients at trial entry according to the Declaration of Helsinki.

### Whole exome capture and nextgen sequence analysis.

Matched diagnostic remission and relapsed DNA samples from 5 ALL patients were used for exome capture with the SureSelect 50 Mb All Exon kit (Agilent Technologies, Santa Clara, CA) following standard protocols. Paired-end sequencing (2 × 100 bp) was carried out by using HiSeq2000 sequencing instruments at Centrillion Biosciences (Mountain View, CA). Illumina HiSeq analysis (Illumina, San Diego, CA) produced between 60 and 120 million paired-end reads per sample. Reads were mapped to the reference genome hgl9 using the Burrows-Wheeler Aligner (BWA) alignment tool version 0.5.9. Mean depth (defined as mean number of reads covering the captured coding sequence of a haploid reference) was 50x with 80% of the genome covered more than lOx (review here) and 57% covered more than 30x. Sites that differ from reference (called here variants) were identified in each sample independently. Empirical priors were constructed for the distribution of variant frequencies for each sample. Credibility intervals were obtained for the change in frequency between tumor and normal samples, using the SAVI algorithm (Statistical Algorithm for Variant Identification) developed at Columbia University (Tiacci, 2011; Pasqualucci, 2011). The number of germline SNPs in the coding region were 18,000 comparable with previous reports (Tiacci, 2011). Most of the candidate germline SNPs (16,000, or -90% of germline variants) were reported in dbSNP database. Candidate somatic variants were identified using the following criteria: variant total depth in tumor and normal larger than lOx and smaller than 300x, variant frequency larger than 15% in tumor and less than 3% in normal, lower bound of the change in frequency in tumor versus normal larger or equal to 1%. Also to remove systematic errors all variants that are present in any of the 23 normal cases were removed. In addition, a 40 base sequence context around the variant were mapped to hgl9 genome using BLAST algorithm to confirm unique mappability (to eliminate ambiguous mapping from captured pseudogenes, and regions of low complexity).

### Mutation validation and analysis of recurrence.

Primers flanking exons containing candidate somatic variants were designed using Primer3 (*see* file *primer3* in subdomain *frodo* of subdomain wi at domain *mit* in category *edu*), and used for PCR amplification from whole-genome-amplified (WGA) tumor, relapse and matched normal (remission) DNAs. The resulting amplicons were analyzed by direct bidirectional dideoxynucleotide sequencing. Mutation recurrence in *TP53, RPL11, BANP* genes and *NT5C2* was analyzed in an independent cohort of 23 relapsed T-ALL patient samples via PCR amplification and bidirectional dideoxinucleotide sequencing. Similarly, the frequency of hotspot *NRAS* activating mutations was analyzed by direct sequencing of PCR products encompassing exons 1 and 2 of the *NRAS* gene.

### Structural depiction and analysis.

Structural coverage of the *NT5C2* protein was identified through use of the PSI-Blast and SKAN algorithms; viable structures were subsequently mapped to all *NT5C2* isoforms, and analyzed with the use of Chimera Suite 3,4. Protein database (PDB) structures 2XCW, 2XCX, 2J2C, 2XCB, 2XCV, 2XJB, 2XJC, 2XJD, 2XJE, 2XJF, 2J2C, and 2JC9 were structurally aligned and the resulting composite structure was subsequently analyzed to assess conformational flexibilities. The identified mutations were structurally modeled utilizing the I-TASSER software suite, and subsequently refined and analyzed via minimization and rotamer library analysis in Chimera3,5. Protein stability changes upon mutation were predicted through use of the SDM potential energy statistical algorithm and associated software6. All structural images were created using UCSF Chimera3.

Biochemical nucleotidase assays measuring release of phosphate upon incubation of *NT5C2* with phosphonucleodides were used, including a commercial kit that measures enzymatic activity. (*See* file *DZ123A.php* in subfolder *reagents* of folder *products* in the World Wide Web at domain *diazyme* in category *com*.)

Table 2 lists mutations identified by exome sequencing in diagnosis and relapse T-ALL. Genomic regions of LOH are available in diagnostic and Libraries of Small Molecules, Peptides and Antibodies. Table 1 lists *NT5C2* mutations in ALL. The correlative clinical data on *NT5C2* mutated relapsed T-ALLs treated in AIEOP clinical trials, and correlative clinical and molecular data on *NT5C2* mutated relapsed T-ALLs in BFM based protocols, and correlative clinical data on rescue treatment for *NT5C2* mutated relapsed T-ALLs in BFM based protocols, are all available. FIG. 9 associates *NT5C2* mutations with clinical characteristics, response, outcome and genetics in T-ALL patients treated in BFM based protocols.

### Site directed mutagenesis.

The *NT5C2* mutations at residues *R291W, K359Q, R367Q* and *D407A* were generated by site directed mutagenesis onto the mammalian expression pLOC-*NT5C2* vector (Open Biosystems/Thermo Scientific of Pittsburgh, Pa.) using the QuickChange II XL Site-Directed Mutagenesis Kit (Stratagene/Agilent Technologies of Santa Clara, Calif.) according to the manufacturer's instructions.

### Cell lines.

CCRF-CEM and CUTLL1 cells were cultured in RPMI-1640 media supplemented with 10% fetal bovine serum, 100U/ml penicillin G and 100ug/ml streptomycin at 37C in a humidified atmosphere under 5% CO2. HEK293T were maintained under similar conditions in DMEM media.

### Lentiviral production and infection.

The lentiviral constructs pLOC-*NT5C2*, pLOC-*NT5C2* 359, pLOC-I 367, pLOC-I 407 and the pLOC-RFP control plasmid were transfected with gag-pol and V-SVG expressing vectors into HEK293T cells using JetPEI transfection reagent (Polyplus, of Illkirch, France). Viral supernatants were collected after 48h and used for infection of CCRF- CEM and CUTLL1 cells by spinoculation. After infection, cells were selected for 5 days in blasticidin and ficolled the day before experiments.

### Cell viability and chemotherapy drug response.

Cell viability was determined by measurement of the metabolic reduction of the tetrazolium salt MTT using the Cell Proliferation Kit I (Roche, of Indianapolis, Ind.) following the manufacturer instructions. Experiments were done in triplicates. Viability was analyzed after 48 or 72 hours of initiation of treatment with 6-mercaptopurine, 6-thioguanine, nelarabine and Ara-G.

### Recombinant protein production and purification.

Full length cDNA constructs encoding wild type *NT5C2, NT5C2 K359Q, NT5C2 R367Q* and *NT5C2 D407A* were cloned with an N-terminal His6 tag in the pET28a-LIC expression vector using the In-Fusion HD PCR cloning system (Clontech, of Mountain View, Calif.), as per manufacturer's instructions. Recombinant proteins were expressed from Rosetta 2(DE3) E. coli cells by induction with 0.5 mM isopropyl-β-D-thiogalactopyranoside for 3 hours at 37°C. Harvested cells were lysed in lysis buffer (50mM sodium phosphate pH7.4, 100mM NaCl, 10% glycerol, 5mM β- mercaptoethanol, 1% Triton X-100, 0.5 mg/ml lysozyme, 20mM imidazole) supplemented with Complete EDTA-free protease inhibitor (Roche, of Indianapolis, IN). His-tagged *NT5C2* proteins were bound to and purified with Nickel-sepharose beads and eluted with 50mM sodium phosphate pH7.4, 100mM NaCl, 10% glycerol, 5mM β-mercaptoethanol, 300mM imidazole. Imidazole was removed by buffer exchange using PD-10 desalting columns (GE Healthcare of Waukesha, Wis.). Protein expression and purity was assessed y SDS-PAGE and Coomassie staining.

### Nucleotidase activity levels.

5'-Nucleotidase activity of purified recombinant wild type and mutant *NT5C2* proteins was assessed using the 5'-nucleotidase (5'-NT) Enzymatic Test Kit (Diazyme, of Poway, CA), according to the manufacturer's instructions. The assay measures the enzymatic hydrolysis of inosine 5'-monophosphate to inosine, which is reacted further to hypoxanthine by purine nucleoside phosphorylase, and then to uric acid and hydrogen peroxide by xanthine oxidase. H2O2 is quantified using a Trinder reaction. 5'-Nucleotidase activity levels are calculated using a calibrator of known 5'-NT activity as standard. Assays were performed in triplicate in an Infinite M200 Tecan (of Mannedorf, Switzerland) plate reader.

### Statistical analysis.

Differences in the percentages of *NT5C2* wild type and mutant ALL patients in different relapsed categories were evaluated using the Fisher's exact test. Equality of categorical and continuous variables was analyzed by Fisher's exact test and by Mann-Whitney U test, respectively.

### Screening Assays

In various embodiments, determining whether the sample includes the biomarker further comprises performing at least one of radioactive or fluorescent DNA sequencing, polymerase chain reaction (PCR), reverse transcription PCR(RTPCR), allele-specific restriction-endonuclease cleavage, allele-specific PCR, allele-specific PCR with suppression of wild type, mismatch-repair detection, binding of MutS protein, denaturing-gradient gel electrophoresis, single-strand-conformation polymorphism detection, RNAase cleavage at mismatched base-pairs, chemical or enzymatic cleavage of heteroduplex DNA, methods based on oligonucleotide-specific primer extension, genetic bit analysis, oligonucleotide-ligation assay, oligonucleotide-specific ligation chain reaction (LCR), gap-LCR, peptide nucleic acid (PNA) assays, Southern Blot analyses, or single stranded conformational polymorphism analyses (SSCP). Assays based on any of these methods are anticipated.

### Allele specific PCR.

Mutant allele detection is based on a modified allele-specific primer, while the wild-type background is suppressed by a proprietary MGB blocker oligonucleotide. Assays can detect less than 0.1% mutant molecules in a background of wild type gDNA, as demonstrated in spiking experiments. Assays demonstrate 7 logs of dynamic range and an average efficiency of 100% ± 10%. A sample is run with one or more assays that target the mutant allele of a given mutation and the corresponding wild type allele assay(s). Three sample replicates are run. This approach is recommended for highly accurate quantitation of the percent mutation in a sample. For data analysis, Mutation Detector™ Software v1.0 allows users to determine the mutation status of their samples from TaqMan®Mutation Detection Assay data collected on Applied Biosystems® ViiA™ Real-Time PCR Systems.

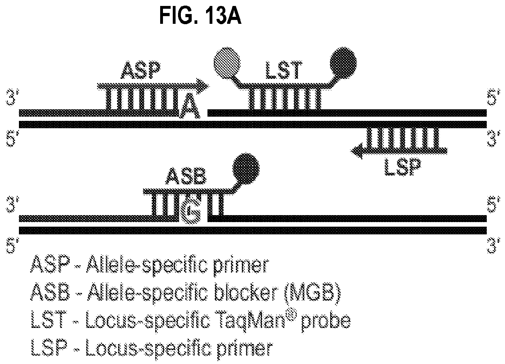

FIG. 13A is a diagram that illustrates an example allele-specific polymerase chain reaction (PCR) with blocker for wild types used to detect genetic variant mutations for *NT5C2,* according to an embodiment. FIG. 13A provides an example to detect a mutant allele comprising a SNP with A at a particular location and a wild type G at that location. The assay components include an allele-specific primer (ASP) to bind to a strand immediately adjacent at the 3' side of the allele, a locus specific probe (LSP) (e.g., a TaqMan probe available from Life Technologies of Grand Island, NY) that binds to the same strand but displaced along the 5' side, and a locus-specific primer LSP that binds to the matching strand displaced further along the 3' end to separate the strands and expose the strand to be assayed. The assay components also include an allele-specific blocker (ASB), such as the proprietary MGB, with a sequence that binds to the wild type strand to inhibit amplification of sequences with the wild type base (e.g., G in the illustration) at the allele position.

For example, to prepare the primers, probes and blockers for the *NT5C2 R367Q* mutation, the gene name as indicated above and the SNP location in the cDNA sequence and nucleotide change, as listed in Table 1, were provided to the vendor. (e.g., Life Technologies of Grand Island, New York)), which generated the various components for this assay including the ASB. In addition, a DNA sequence of 200 bp flanking each side of the SNP in genomic DNA with the nucleotide change indicated in brackets was also sent. Thus, NT5C2 R367Q castPCR design: Single nucleotide change with cDNA location Ref. Seq NM_012229 1100G>A. The assay is designed for detection in genomic DNA samples. Genomic region encompassing the mutation is: >Human_NT5C2_R367Q, (SEQ. ID NO. 9) in which intron sequences are shown in lower case and exon sequences in upper case.

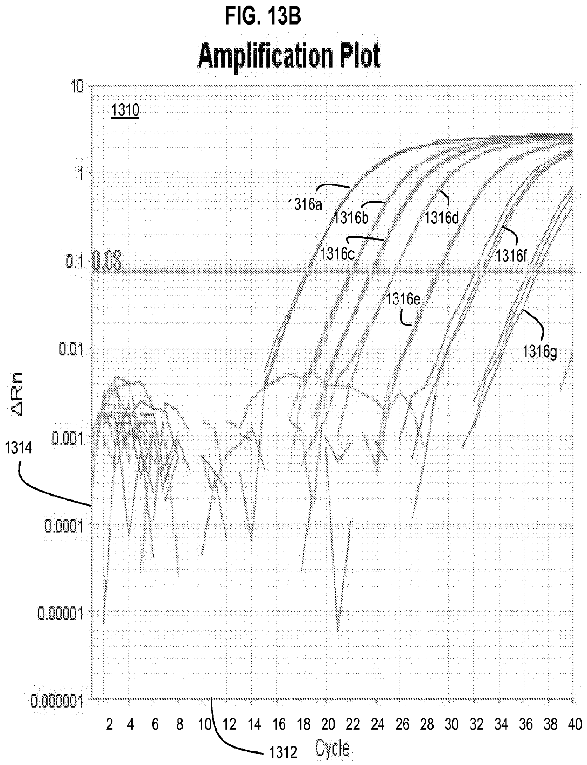

FIG. 13B is a graph 1310 that illustrates an example amplification of a genetic variant for *NT5C2* using the allele specific PCR process of FIG. 13A, according to an embodiment. Here allele specific PCR is used to detect the one mutation *NT5C2 R367Q.* Rn is the fluorescence of the reporter dye divided by the fluorescence of a passive reference dye; e.g., Rn is the reporter signal normalized to the fluorescence signal of ROX™. ΔRn is Rn minus the baseline; ΔRn is plotted against PCR cycle number. An amplification plot shows the variation of log (ΔRn) on vertical axis 1314 with PCR cycle number on the horizontal axis 1312. Three runs are made on each sample, and the results from 7 different samples are shown as trace groups 1316a, 1316b, 1316c, 1316d, 1316e, 1316f and 1316g. The first few cycles are dominated by noise in the optical measurements, but by cycle 16 some samples have achieved measureable fluorescence, and by cycle 35 all samples have exceeded a reliability threshold of ΔRn = 0.08. The difference among samples is due to different amounts of the target allele *NT5C2 R367Q* in each sample.

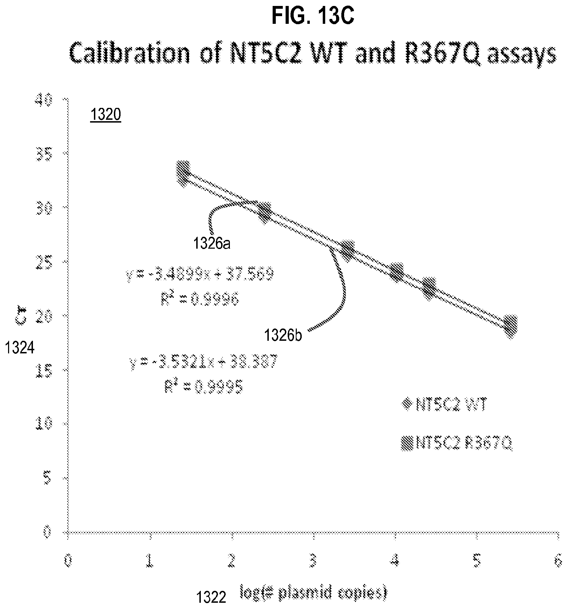

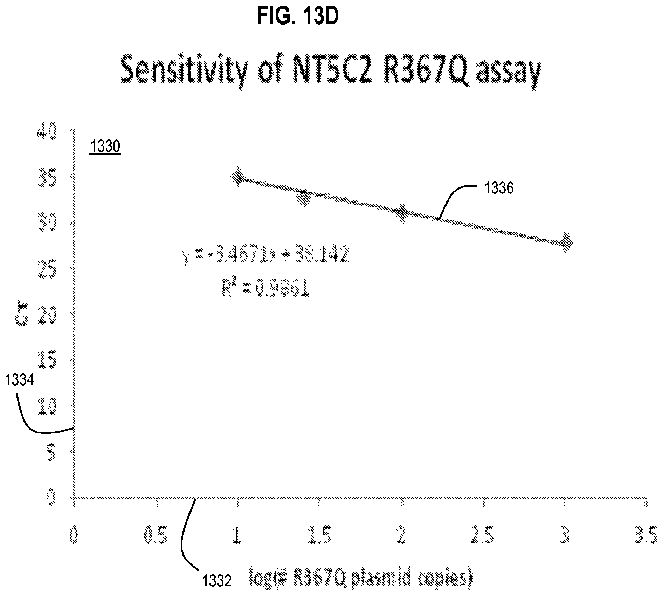

FIG. 13C and FIG. 13D are graphs 1320, 1330, respectively, that illustrate example calibration and sensitivity, respectively, of one genetic variant mutation for *NT5C2* using the allele specific PCR process of FIG. 13A, according to an embodiment. The calibration plot 1320 includes a horizontal axis (x) 1322 that indicates a log of the number of plasmid copies (dimensionless) included in a sample, and a vertical axis (y) 1324 that indicates C_{T}, the cycle at which a threshold signal is reached (e.g,, a threshold of ΔRn = 0.08, as depicted in FIG. 13B). The trace 1326a shows calibration curve for genes for the wild type protein and trace 1326b shows the calibration curve genes for the target mutation. The calibration equations are also shown. The calibration curves are used to determine the number of plasmid copies in a sample based on the cycle on which the PCR fluorescence signal reaches the threshold value. The sensitivity plot 1330 includes a horizontal axis 1332 that indicates a log of the number of plasmid copies (dimensionless) of the mutations *NT5C2 R367Q* included in a sample, and a vertical axis 1334 that indicates C_{T}, the cycle at which a threshold signal is reached. The trace 1336 shows the sensitivity curve genes for the target mutation. The sensitivity equation is also shown. The sensitivity curve is used to determine the smallest change in plasmid amount that can be detected with a change in the number of cycles. With a change of 2 in log (e.g., a factor of 100) the number of cycles differ by about 7, thus each cycle indicates a change in amount of about 0.3 in log values, corresponding to a factor of about 2 in the number of mutations present.

### Northern Analysis.

Northern analysis remains the standard for detection and quantitation of mRNA levels despite the advent of more sensitive techniques. Northern analysis presents several advantages over the other techniques. The most compelling of these is that it is the easiest method for determining transcript size, and for identifying alternatively spliced transcripts and multigene family members. It can also be used to directly compare the relative abundance of a given message between all the samples on a blot. The Northern blotting procedure is straightforward and provides opportunities to evaluate progress at various points (e.g., intactness of the RNA sample and how efficiently it has transferred to the membrane). RNA samples are first separated by size via electrophoresis in an agarose gel under denaturing conditions. The RNA is then transferred to a membrane, crosslinked and hybridized with a labeled probe. Nonisotopic or high specific activity radiolabeled probes can be used including random-primed, nick-translated, PCR-generated DNA probes, in vitro transcribed RNA probes, and oligonucleotides. Additionally, sequences with only partial homology (e.g., cDNA from a different species or genomic DNA fragments that might contain an exon) may be used as probes. Despite these advantages, there are limitations associated with Northern analysis. First, if RNA samples are even slightly degraded, the quality of the data and the ability to quantitate expression are severely compromised. Second, a standard Northern procedure is, in general, the least sensitive of several popular techniques. However, substantial improvements can be made to increase detection of most mRNA species (see below). Another limitation of Northern blotting has been the difficulty associated with multiple probe analysis. To detect more than one message, it is usually necessary to strip the initial probe before hybridizing with a second probe. This process can be time consuming and problematic.

### Nuclease Protection Assay (NPA).

The NPA (including both ribonuclease protection assays and S1 nuclease assays) is an extremely sensitive method for the detection and quantitation of specific mRNAs. The basis of the NPA is solution hybridization of an antisense probe (radiolabeled or nonisotopic) to an RNA sample. After hybridization, single-stranded, unhybridized probe and RNA are degraded by nucleases. The remaining protected fragments are separated on an acrylamide gel. Solution hybridization is typically more efficient than membrane-based hybridization, and it can accommodate up to 100 µg of sample RNA, compared with the 20-30 µg maximum of blot hybridizations. NPAs are also less sensitive to RNA sample degradation than Northern analysis since cleavage is only detected in the region of overlap with the probe (probes are usually about 100-400 bases in length). NPAs are the method of choice for the simultaneous detection of several RNA species. At least 6 different probes and an internal control can be used together for detection of mRNA transcripts each within 4 different RNA samples. During solution hybridization and subsequent analysis, individual probe/target interactions are completely independent of one another. Thus, several RNA targets and appropriate controls can be assayed simultaneously (up to twelve have been used in the same reaction), provided that the individual probes are of different lengths. NPAs are also commonly used to precisely map mRNA termini and intron/exon junctions. The primary limitation of NPAs is the lack of information on transcript size. The portion of probe homologous to target RNA determines the size of the protected fragment. Another drawback to NPAs is the lack of probe flexibility. The most common type of NPA, the ribonuclease protection assay, requires the use of RNA probes. Oligonucleotides and other single-stranded DNA probes can only be used in assays containing S1 nuclease. The single-stranded, antisense probe must typically be completely homologous to target RNA to prevent cleavage of the probe:target hybrid by nuclease. This means that partially related sequences (i.e., cross species) usually cannot be used.

### RNA Analysis in a Single Tube.

Ambion (of Austin, TX) released the first commercially available Ribonuclease Protection Assay Kit in 1990 and has since continued to refine and improve the procedure resulting in a number of kits with different advantages. The RPA III™ Kit is recommended for all new users and consists of a single-tube protocol. The HybSpeed™ RPA Kit utilizes a 10-minute hybridization step so that the entire assay can be completed in a single day.

### In Situ Hybridization (ISH).

In situ hybridization (ISH) is a powerful and versatile tool for the localization of specific mRNAs in cells or tissues. Unlike Northern analysis and nuclease protection assays, ISH does not require the isolation or electrophoretic separation of RNA. Hybridization of the probe takes place within the cell or tissue. Since cellular structure is maintained throughout the procedure, ISH provides information about the location of mRNA within the tissue sample. The procedure begins by fixing samples in neutral-buffered formalin, and embedding the tissue in paraffin. The samples are then sliced into thin sections and mounted onto microscope slides. (Alternatively, tissue can be sectioned frozen and post-fixed in paraformaldehyde.) After a series of washes to dewax and rehydrate the sections, a Proteinase K digestion is performed to increase probe accessibility, and a labeled probe is then hybridized to the sample sections. Radiolabeled probes are visualized with liquid film dried onto the slides, while nonisotopically labeled probes are conveniently detected with colorimetric or fluorescent reagents. The major drawback to ISH is the procedure itself. Standard protocols are time-consuming, laborious and may require specialized equipment for preparing samples and visualizing results of the experiment. Additionally, quantitation of gene expression is not as straightforward as with the other techniques.

### RT-PCR.

RT-PCR has revolutionized the study of gene expression. It is now theoretically possible to detect the RNA transcript of any gene, regardless of the scarcity of the starting material or relative abundance of the specific mRNA. In RT-PCR, an RNA template is copied into a complementary DNA (cDNA) using a retroviral reverse transcriptase. The cDNA is then amplified exponentially by PCR. As with NPAs, RT-PCR is somewhat tolerant of degraded RNA. As long as the RNA is intact within the region spanned by the primers, the target will be amplified. Although RT-PCR is the most sensitive method of mRNA detection available, it does have drawbacks. It can be the most technically challenging method of detection and quantitation, often requiring substantial pre-experimental planning and design. Additionally, because of its extreme sensitivity, even minute amounts of contamination by genomic DNA or previously amplified PCR products can lead to aberrant results, so steps must be taken to avoid this pitfall. Relative quantitative RT-PCR involves amplifying an internal control simultaneously with the gene of interest. The internal control is used to normalize the samples. Once normalized, direct comparisons of relative abundance of a specific mRNA can be made across the samples. It is crucial to choose an internal control with a constant level of expression across all experimental samples (i.e., not affected by experimental treatment). Commonly used internal controls (e.g., GAPDH, -actin, cyclophilin) often vary in expression and, therefore, may not be appropriate internal controls. Additionally, most common internal controls are expressed at much higher levels than the mRNA being studied. For relative RT-PCR results to be meaningful, all products of the PCR reaction must be analyzed in the linear range of amplification. This becomes difficult for transcripts of widely different levels of abundance.

### Competitive RT-PCR.

This is used for absolute quantitation. This technique involves designing, synthesizing, and accurately quantitating a competitor RNA that can be distinguished from the endogenous target by a small difference in size or sequence. Known amounts of the competitor RNA are added to experimental samples and RT-PCR is performed. Signals from the endogenous target are compared with signals from the competitor to determine the amount of target present in the sample. Ambion (Austin, TX) has a complete line of products to simplify RT-PCR. The RETROscript™ Kit provides a convenient and flexible system for synthesizing first-strand cDNA. The QuantumRNA™ 18S Internal Standards Kits utilize Ambion's Competimer™ technology (patent pending) to allow the use of the best internal control, 18S rRNA, for relative RT-PCR (Figure 3). As detailed above, there are several methods for detecting and quantitating mRNA, each with advantages and disadvantages. Northern analysis provides information about the endogenous transcript size and is tolerant of many types of probe. The NPA is a simple format that is ideal for multi-target analysis and mapping studies. Gene expression can be localized within tissue or cells using in situ hybridization, and even the rarest messages can be detected with RT-PCR. Deciding which technique to use will depend on exactly what information is needed. Because no single method provides all of the information, a thorough analysis of gene expression often requires the use of multiple techniques.

### Ligand assays.

Some embodiments are directed to a method for screening and identifying test compounds (ligands) that bind to a preselected target protein. Direct, non-competitive binding assays are advantageously used to screen libraries of compounds (such as bead-based libraries) for those that selectively bind to a preselected target. Binding of target molecules to a particular test compound is detected using any physical method known in the art for detecting high affinity binding of the ligand to the target protein. The structure of the test compound (ligand) that binds to the target can also be determined, if it is not known beforehand. The methods used will depend, in part, on the nature of the library screened. The methods of the present invention provide a simple, sensitive assay for high-throughput screening of libraries of compounds to identify ligands that bind with high affinity to the target *NT5C2* protein.

For example, in some embodiments, a screening assay for identifying a ligand that binds with high affinity to *NT5C2* protein or to a biologically active fragment or variant thereof, includes screening a ligand library against *NT5C2* or a biologically active fragment or variant thereof to identify a ligand that binds with high affinity to the *NT5C2* protein. The assay includes isolating the high affinity ligand from the library. Then the high affinity ligand is combined with the *NT5C2* protein and the nucleoside analog under conditions that permit the *NT5C2* protein to bind to the nucleoside analog. The assay includes determining whether the *NT5C2* protein binds to the nucleoside analog in a mixture forming a *NT5C2* protein-nucleoside complex. If not, then the assay includes selecting and identifying the high affinity ligand as one that prevents *NT5C2* protein from inactivating the nucleoside analog.

In some embodiments, the assay also includes combining the high affinity ligand, the *NT5C2* protein and the nucleoside analog under conditions that permit the nucleoside analog to produce active metabolites in a cell. This assay also includes measuring for the presence or absence of the active metabolites in the cell if the *NT5C2* protein-nucleoside complex is not formed. If the active metabolites of nucleoside analogs are present in normal amounts in the cell, then it is determined that the high affinity ligand prevents the expulsion of metabolites of conventional nucleoside analog therapy and is suitable for including in a treatment for a patient with the *NT5C2* mutations. In some of these embodiments, the metabolites are selected from the group consisting of TIMP, TXMP and TGMP.

As used herein, a "library" refers to a plurality of test compounds (protein, antibody, small molecules) with which a target protein is contacted. A library can be a combinatorial library, *e.g.,* a collection of test compounds synthesized using combinatorial chemistry techniques, or a collection of unique chemicals of low molecular weight (less than 1000 daltons) that each occupy a unique three-dimensional space. As used herein, a "label" or "detectable label" is a composition that is detectable, either directly or indirectly, by spectroscopic, photochemical, biochemical, immunochemical, or chemical means.

Methods are described in which a preselected target protein having a detectable label is used to screen a library of test compounds. Any complexes formed between the target and a member of the library are identified using methods that detect the target bound to a test compound. In an embodiment, the present invention relates to methods for using a target (optionally having a detectable label) to screen a library of test compounds. Compounds in the library that bind to the target will form a complex, which can be separated from the unbound target. The complex can then be identified and removed from the uncomplexed target and uncomplexed compounds.

In an embodiment, the protein is bound to a support and the test compound is free. In some embodiments the target is labeled, for example with a fluorescent dye, phosphorescent dye, ultraviolet dye, infrared dye, visible radiolabel, enzyme, spectroscopic colorimetric label, affinity tag, or nanoparticle. In an embodiment, a combinatorial library of test compounds is used comprising peptoids; polypeptides; nonpeptidal peptidominetics; oligocarbamates; antibody libraries; carbohydrate libraries; and organic molecule libraries.

Libraries screened using the methods of the present invention can comprise a variety of types of test compounds, optionally bound to solid supports. In all of the embodiments described below, all of the libraries can be synthesized on solid supports or the compounds of the library can be attached to solid supports by linkers, or they can be in solution.

In some embodiments, the test compounds are peptide molecules that can exist in a phage display library. In other embodiments, types of test compounds include, but are not limited to, peptide analogs including peptides comprising non-naturally occurring amino acids, *e.g.,* D-amino acids, phosphorous analogs of amino acids, such as α-amino phosphoric acids and α-amino phosphoric acids, or amino acids having non-peptide linkages, nucleic acid analogs such as phosphorothioates and PNAs, hormones, antigens, synthetic or naturally occurring drugs, opiates, dopamine, serotonin, catecholamines, thrombin, acetylcholine, prostaglandins, organic molecules, pheromones, adenosine, sucrose, glucose, lactose and galactose. Libraries of polypeptides or proteins can also be used.

In a preferred embodiment, the combinatorial libraries are small organic molecule libraries, such as, but not limited to, benzodiazepines, isoprenoids, thiazolidinones, metathiazanones, pyrrolidines, morpholino compounds, and diazepindiones. In another embodiment, the combinatorial libraries comprise peptoids; random bio-oligomers; benzodiazepines; diversomers such as hydantoins, benzodiazepines and dipeptides; vinylogous polypeptides; nonpeptidal peptidomimetics; oligocarbamates; peptidyl phosphonates; peptide nucleic acid libraries; antibody libraries; or carbohydrate libraries. Combinatorial libraries are themselves commercially available (*see, e.g.,* Advanced ChemTech Europe Ltd., Cambridgeshire, UK; ASINEX, Moscow, Russia; Bifocals plc, Sittingbourne, UK; Bionet Research [a division of Key Organics Limited], Camelford, UK; ChemBridge Corporation, San Diego, Calif.; ChemDiv Inc, San Diego, Calif.; ChemRx Advanced Technologies, South San Francisco, Calif.; ComGenex Inc., Budapest, Hungary; Evotec OAI Ltd, Abingdon, UK; IF LAB Ltd., Kiev, Ukraine; Maybridge plc, Cornwall, UK; PharmaCore, Inc., High Point, N.C.; SIDDCO Inc, Tucson, Ariz.; TimTec Inc, Newark, Del.; Tripos Receptor Research Ltd, Bude, UK; Toslab, Ekaterinburg, Russia).

In one teaching, a combinatorial compound library for use with the present invention may be synthesized. Combinatorial compound libraries useful for the methods of the present invention can be synthesized on solid supports. As used herein, the term "solid support" is not limited to a specific type of solid support. Rather a large number of supports are available and are known to one skilled in the art. Solid supports include silica gels, resins, derivatized plastic films, glass beads, cotton, plastic beads, polystyrene beads, doped polystyrene beads (as described by Fenniri, 2001), alumina gels, and polysaccharides. A suitable solid support may be selected on the basis of desired end use and suitability for various synthetic protocols. In some embodiments of the present invention, compounds can be attached to solid supports via linkers.

Screening comprises contacting a target with an individual or small group, of the components of the compound library. Preferably, the contacting occurs in an aqueous solution, and most preferably, under physiologic conditions. The aqueous solution preferably stabilizes the target and prevents denaturation or degradation without interfering with binding of the test compounds.

The methods of some teachings for screening a library of test compounds preferably comprise contacting a test compound with a target in the presence of an aqueous solution, the aqueous solution comprising a buffer and a combination of salts, preferably approximating or mimicking physiologic conditions.

As used herein, a "dye" refers to a molecule that, when exposed to radiation, emits radiation at a level that is detectable visually or via conventional spectroscopic means. As used herein, a "visible dye" refers to a molecule having a chromophore that absorbs radiation in the visible region of the spectrum (*i*.*e*., having a wavelength of between about 400 nm and about 700 nm) such that the transmitted radiation is in the visible region and can be detected either visually or by conventional spectroscopic means. As used herein, an "ultraviolet dye" refers to a molecule having a chromophore that absorbs radiation in the ultraviolet region of the spectrum (*i.e*., having a wavelength of between about 30 nm and about 400 nm). As used herein, an "infrared dye" refers to a molecule having a chromophore that absorbs radiation in the infrared region of the spectrum (*i*.*e*., having a wavelength between about 700 nm and about 3,000 nm). A "chromophore" is the network of atoms of the dye that, when exposed to radiation, emits radiation at a level that is detectable visually or via conventional spectroscopic means. One of skill in the art will readily appreciate that although a dye absorbs radiation in one region of the spectrum, it may emit radiation in another region of the spectrum. For example, an ultraviolet dye may emit radiation in the visible region of the spectrum. One of skill in the art will also readily appreciate that a dye can transmit radiation or can emit radiation via fluorescence or phosphorescence.

As used herein, a "label" or "detectable label" is a composition that is detectable, either directly or indirectly, by spectroscopic, photochemical, biochemical, immunochemical, or chemical means. For example, useful labels include radioactive isotopes (*e.g.*, ³²P, ³⁵S, and ³H), dyes, fluorescent dyes, electron-dense reagents, enzymes and their substrates (*e.g*., as commonly used in enzyme-linked immunoassays, *e.g.,* alkaline phosphatase and horseradish peroxidase), biotin, digoxigenin, or haptens and proteins for which antisera or monoclonal antibodies are available. Moreover, a label or detectable moiety can include an "affinity tag" that, when coupled with the target nucleic acid and incubated with a test compound or compound library, allows for the affinity capture of the target nucleic acid along with molecules bound to the target nucleic acid. One skilled in the art will appreciate that a affinity tag bound to the target nucleic acids has, by definition, a complimentary ligand coupled to a solid support that allows for its capture. For example, useful affinity tags and complimentary ligands include, but are not limited to, biotin-streptavidin, complimentary nucleic acid fragments (*e.g*., oligo dot-oligo da, oligo T-oligo A, oligo dig-oligo do, oligo G-oligo C), aptamer complexes, or haptens and proteins for which antisera or monoclonal antibodies are available. The label or detectable moiety is typically bound, either covalently, through a linker or chemical bound, or through ionic, van der Waals or hydrogen bonds to the molecule to be detected.

### Kits

Some embodiments comprise a kit for detecting the presence of a biomarker in a biological sample selected from the group consisting of a *NT5C2* gene mutation, an mRNA transcribed from the *NT5C2* gene mutation, or a protein encoded by the *NT5C2* gene mutation in a biological sample. The kit includes reagents for detecting the presence of the *NT5C2* gene mutation, the mRNA transcribed from the *NT5C2* gene mutation, or the protein encoded by the *NT5C2* gene mutation, and instructions for use of the kit to determine if a subject has increased resistance to treatment with 6-mercaptopurine or 6-thioguanine.
In some embodiments the kit includes one or more control samples. Examples of negative control samples are: wild type DNA, RNA or NT5C2 protein. Examples of positive control samples include: mutant NT5C2 DNA, RNA and proteins. Example, calibration and sensitivity controls include: mutant NT5C2 DNA, RNA and protein diluted in wild type NT5C2 DNA, RNA and proteins at known ratios.

### Extensions, alterations and modifications

In the present specification, the invention has been described with reference to specific embodiments thereof. The specification and drawings are to be regarded in an illustrative rather than a restrictive sense. Although specific terms are employed, they are used as in the art unless otherwise indicated.

### REFERENCES

Altschul, S.F., et al. Gapped BLAST and PSI-BLAST: a new generation of protein database search programs. Nucleic Acids Res 25, 3389-3402 (1997).
Berg, S.L. et al. Phase II study of nelarabine (compound 506U78) in children and young adults with refractory T-cell malignancies: a report from the Children's Oncology Group. J Clin Oncol 23, 3376-3382 (2005).
Bhatia, S., et al. Nonadherence to oral mercaptopurine and risk of relapse in Hispanic and non-Hispanic white children with acute lymphoblastic leukemia: a report from the children's oncology group. J Clin Oncol 30, 2094-2101 (2012).
Bowden, D.H., Adamson, I.Y. & Wyatt, J.P. Reaction of the lung cells to a high concentration of oxygen. Arch Pathol 86, 671-675 (1968).
Brouwer, C., et al. Role of 5'-nucleotidase in thiopurine metabolism: enzyme kinetic profile and association with thio-GMP levels in patients with acute lymphoblastic leukemia during 6-mercaptopurine treatment. Clin Chim Acta 361, 95-103 (2005).
Bunz, F., et al. Disruption of p53 in human cancer cells alters the responses to therapeutic agents. J Clin Invest 104, 263-269 (1999).
Clappier, E., et al. Clonal selection in xenografted human T cell acute lymphoblastic leukemia recapitulates gain of malignancy at relapse. J Exp Med 208, 653-661 (2011).
Cros, E., Jordheim, L., Dumontet, C. & Galmarini, C.M. Problems related to resistance to cytarabine in acute myeloid leukemia. Leuk Lymphoma 45, 1123-1132 (2004).
DeAngelo, D.J., et al. Nelarabine induces complete remissions in adults with relapsed or refractory T-lineage acute lymphoblastic leukemia or lymphoblastic lymphoma: Cancer and Leukemia Group B study 19801. Blood 109, 5136-5142 (2007).
Fenniri, H., et al. Barcoded Resins: A New Concept for Polymer-Supported Combinatorial Library Self-Deconvolution. J. Am. Chem. Soc. 123, 8151-8152 (2001).
Galmarini, C.M., et al. Deoxycytidine kinase and cN-II nucleotidase expression in blast cells predict survival in acute myeloid leukaemia patients treated with cytarabine. Br J Haematol 122, 53-60 (2003).
Galmarini, C.M., Jordheim, L. and Dumontet, C. Role of IMP-selective 5'-nucleotidase (cN-II) in hematological malignancies. Leuk Lymphoma 44, 1105-1111 (2003a).
Galmarini, C.M., Cros, E., Thomas, X., Jordheim, L. & Dumontet, C. The prognostic value of cN-II and cN-III enzymes in adult acute myeloid leukemia. Haematologica 90, 1699-1701 (2005).
Gokbuget, N., et al. High single-drug activity of nelarabine in relapsed T-lymphoblastic leukemia/lymphoma offers curative option with subsequent stem cell transplantation. Blood 118, 3504-3511 (2011).
Hof, J., et al. Mutations and deletions of the TP53 gene predict nonresponse to treatment and poor outcome in first relapse of childhood acute lymphoblastic leukemia. J Clin Oncol 29, 3185-3196 (2011).
Hunsucker, S.A., Mitchell, B.S. and Spychala, J. The 5'-nucleotidases as regulators of nucleotide and drug metabolism. Pharmacol Ther 107, 1-30 (2005).
Jordheim, L., Galmarini, C.M. & Dumontet, C. Drug resistance to cytotoxic nucleoside analogues. Curr Drug Targets 4, 443-460 (2003).
Jordheim, L., et al. Identification and characterization of inhibitors of cytoplasmic 5'-nucelotidase cN-II issued from virtual screening. Biochem Pharm 85, pp 497-506 (2013).
McCubrey, J.A., et al. Roles of the Raf/MEK/ERK pathway in cell growth, malignant transformation and drug resistance. Biochem Biophys Acta 1773, 1263-1284 (2007).
Mullighan, C.G., et al. Genomic analysis of the clonal origins of relapsed acute lymphoblastic leukemia. Science 322, 1377-1380 (2008).
Oka, J., Matsumoto, A., Hosokawa, Y. & Inoue, S. Molecular cloning of human cytosolic purine 5'-nucleotidase. Biochem Biophys Res Commun 205, 917-922 (1994).
Pasqualucci, L., et al. Analysis of the coding genome of diffuse large B-cell lymphoma. Nat Genet 43, 830-837 (2011).
Pettersen, E.F., et al. UCSF Chimera--a visualization system for exploratory research and analysis. J Comput Chem 25, 1605-1612 (2004).
Pieters, R., et al. Relation of 5'-nucleotidase and phosphatase activities with immunophenotype, drug resistance and clinical prognosis in childhood leukemia. Leuk Res 16, 873-880 (1992).
Roy, A., Kucukural, A. & Zhang, Y. I-TASSER: a unified platform for automated protein structure and function prediction. Nat Protoc 5, 725-738.
Sanford, M. & Lyseng-Williamson, K.A. Nelarabine. Drugs 68, 439-447 (2008).
Spychala, J., Madrid-Marina, V. & Fox, I.H. High Km soluble 5'-nucleotidase from human placenta. Properties and allosteric regulation by IMP and ATP. J. Biol. Chem. 263, 18759-18765 (1988).
Thompson, B.J., et al. The SCFFBW7 ubiquitin ligase complex as a tumor suppressor in T cell leukemia. J Exp Med 204, 1825-1835 (2007).
Tiacci, E., et al. BRAF mutations in hairy-cell leukemia. N Engl J Med 364, 2305-2315 (2011).
Tosello, V., et al. WT1 mutations in T-ALL. Blood 114, 1038-1045 (2009).
Wallden, K. & Nordlund, P. Structural basis for the allosteric regulation and substrate recognition of human cytosolic 5'-nucleotidase II. J Mol Biol 408, 684-696 (2011).
Worth, C.L., Preissner, R. & Blundell, T.L. SDM-a server for predicting effects of mutations on protein stability and malfunction. Nucleic Acids Res 39, W215-222 (2011).
Zhang, F., et al. Ribosomal stress couples the unfolded protein response to p53-dependent cell cycle arrest. J Biol Chem 281, 30036-30045 (2006).
Zhang, J., et al. The genetic basis of early T-cell precursor acute lymphoblastic leukaemia. Nature 481, 157-163 (2012).

## Claims

1. A nucleoside analog selected from 6-mercaptopurine, 6-thioguanine and nelarabine for use in a method of treating of a subject with acute lymphoblastic leukemia, wherein
(a) if the subject does not have the presence of a detectable *NT5C2* gene mutation which results in an associated protein mutation selected from the group consisting of R238W, R238L, R291W, K359Q, S360P, R367Q, L375F, D407A, D407Y and Q523*, the nucleoside analog is 6-mercaptopurine or 6-thioguanine; and
(b) if the subject has the presence of a detectable *NT5C2* gene mutation which results in an associated protein mutation selected from the group consisting of R238W, R238L, R291W, K359Q, S360P, R367Q, L375F, D407A, D407Y and Q523*, the nucleoside analog is nelarabine.

2. A nucleoside analog for use in a method as recited in claim 1, wherein the presence of the *NT5C2* gene mutation is detectable using an allele-specific polymerase chain reaction (PCR) assay;
wherein * represents a mutation to a STOP codon.

3. A nucleoside analog selected from 6-mercaptopurine, 6-thioguanine and nelarabine for use in a method as recited in claim 1, wherein the presence of the *NT5C2* gene mutation is detectable by performing at least one of radioactive or fluorescent DNA sequencing, polymerase chain reaction (PCR), reverse transcription PCR(RTPCR), allele-specific restriction-endonuclease cleavage, mismatch-repair detection, binding of MutS protein, denaturing-gradient gel electrophoresis, single-strand-conformation polymorphism detection, RNAase cleavage at mismatched base-pairs, chemical or enzymatic cleavage of heteroduplex DNA, methods based on oligonucleotide-specific primer extension, genetic bit analysis, oligonucleotide-ligation assay, oligonucleotide-specific ligation chain reaction (LCR), gap-LCR, peptide nucleic acid (PNA) assays, Southern Blot analyses, single stranded conformational polymorphism analyses (SSCP), deoxyribonucleic acid sequencing, restriction fragment length polymorphism (RFLP) analysis.

4. A nucleoside analog selected from 6-mercaptopurine, 6-thioguanine and nelarabine for use in a method as recited in claim 1, wherein the presence of the *NT5C2* gene mutation is detectableusing a method from the group comprising Northern blot analysis, nuclease protection assays (NPA), in situ hybridization, and reverse transcription-polymerase chain reaction (RT-PCR) mRNA sequencing, or wherein detecting the biomarker selected from the protein encoded by the *NT5C2* gene mutation comprises using a method from the group comprising immunostaining, immunoprecipitation, immunoelectrophoresis, Immunoblotting, BCA assay to quantify protein concentrations), Western blot, Spectrophotometry, and enzyme assays.

5. The nucleoside analog selected from 6-mercaptopurine, 6-thioguanine and nelarabine for use in a method as recited in of claim 1, wherein the presence of the NT5C2 gene mutation is detectable in a biological sample from the subject which is selected from the group consisting of serum, blood, peripheral blood, plasma, blood cells, pleural effusion, cerebrospinal fluid, bone marrow or tissue biopsy.

6. A kit for use in detecting in a biological sample the presence of a detectable *NT5C2* gene mutation which results in an associated protein mutation selected from the group consisting of R238W, R238L, R291W, K359Q, S360P, R367Q, L375F, D407A, D407Y and Q523*, the kit comprising reagents for detecting the presence said *NT5C2* gene mutation, and instructions for use of the kit, wherein * represents a mutation to a STOP codon.

7. The kit for use of claim 6, further comprising means for obtaining a biological sample from a subject.

8. The kit for use of claim 6, further comprising a control sample.

9. The kit for use of claim 6, which comprises the means for assaying SNPs is allele-specific PCR and the kit comprises one or more of the following: allele specific primer, allele specific blocker, a locus-specific TaqMan probe, and a locus-specific primer.

## Patentansprüche

1. Nucleosidanalogon, das ausgewählt ist aus 6-Mercaptopurin, 6-Thioguanin und Nelarabin für die Verwendung in einem Verfahren zum Behandeln eines Subjekts mit akuter lymphatischer Leukämie, wobei
(a) wenn das Subjekt nicht das Vorhandensein einer detektierbaren *NT5C2-*Genmutation aufweist, die eine assoziierte Proteinmutation zur Folge hat, ausgewählt aus der Gruppe bestehend aus R238W, R238L, R291W, K359Q, S360P, R367Q, L375F, D407A, D407Y und Q523*, das Nucleosidanalogon dann 6-Mercaptopurin oder 6-Thioguanin ist; und
(b) wenn das Subjekt das Vorhandensein einer detektierbaren *NT5C2-*Genmutation aufweist, die eine assoziierte Proteinmutation zur Folge hat, die ausgewählt ist aus der Gruppe bestehend aus R238W, R238L, R291W, K359Q, S360P, R367Q, L375F, D407A, D407Y und Q523*, das Nucleosidanalogon dann Nelarabin ist.

2. Nucleosidanalogon zur Verwendung in einem Verfahren nach Anspruch 1, wobei das Vorhandensein der *NT5C2*-Genmutation detektierbar ist, indem ein Allel-spezifischer Polymerase-Kettenreaktions (PCR)-Assay angewendet wird,
wobei * eine Mutation zu einem Stoppcodon darstellt.

3. Nucleosidanalogon, das ausgewählt ist aus 6-Mercaptopurin, 6-Thioguanin und Nelarabin für die Verwendung in einem Verfahren nach Anspruch 1, wobei das Vorhandensein der *NT5C2*-Genmutation detektierbar ist, indem mindestens eine der Folgenden ausgeführt wird: radioaktives oder fluoreszierendes DNA-Sequenzieren, Polymerasekettenreaktion (PCR), reverse Transkription PCR (RTPCR), Allel-spezifische Restriktionsendonuclease-Spaltung, Fehlpaarungsreparatur-Detektion, Binden von MutS-Protein, Denaturierungsgradientenelelektrophorese, Detektion von Einzelstrang-Konformations-Polymorphismus, RNAase-Spaltung an falsch gepaarten Basenpaaren, chemische oder enzymatische Spaltung von Heteroduplex-DNA, Verfahren, die auf Oligonucleotid-spezifischer Primerextension basieren, genetische Bitanalyse, Oligonucleotid-Ligationsassay, Oligonucleotid-spezifische Ligationskettenreaktion (LCR), Gap-LCR, Assays von Peptidnucleinsäure (PNA), Southern Blot-Analysen, Analysen von Einzelstrang-Konformations-Polymorphismus (SSCP), Sequenzieren von Desoxyribonucleinsäure, Analyse von Restriktions-Fragmentlängen-Polymorphismus (RFLP).

4. Nucleosidanalogon, das ausgewählt ist aus 6-Mercaptopurin, 6-Thioguanin und Nelarabin für die Verwendung in einem Verfahren nach Anspruch 1, wobei das Vorhandensein der *NT5C2*-Genmutation detektierbar ist unter Verwendung eines der Verfahren aus der Gruppe, umfassend: Northern Blot-Analyse, Nuclease-Protection-Assays (NPA), In-situ-Hybridisierung und Sequenzieren von reverser Transkriptions-Polymerasekettenreaktion (RT-PCR) mRNA, oder wobei das Detektieren des Biomarkers, der ausgewählt ist aus dem Protein, das durch die *NT5C2*-Genmutation codiert wird, das Anwenden eines Verfahrens aus der Gruppe umfasst, umfassend Immunfärben, Immunpräzipitation, Immunelektrophorese, Immunoblotting, BCA-Assay, um Konzentrationen von Protein zu quantifizieren, Western Blot, Spektrophotometrie und Enzym-Assays.

5. Nucleosidanalogon, das ausgewählt ist aus 6-Mercaptopurin, 6-Thioguanin und Nelarabin für die Verwendung in einem Verfahren nach Anspruch 1, wobei das Vorhandensein der NT5C2-Genmutation in einer biologischen Probe des Subjekts detektierbar ist, die ausgewählt ist aus der Gruppe bestehend aus Serum, Blut, peripherem Blut, Plasma, Blutzellen, Pleura-Erguss, Cerebrospinalflüssigkeit, Knochenmark oder Gewebebiopsie.

6. Kit zur Verwendung beim Detektieren in einer biologischen Probe des Vorhandenseins einer detektierbaren *NT5C2*-Genmutation, die eine assoziierte Proteinmutation zur Folge hat, die ausgewählt ist aus der Gruppe bestehend aus R238W, R238L, R291W, K359Q, S360P, R367Q, L375F, D407A, D407Y und Q523*, wobei das Kit Reagenzien zum Detektieren des Vorhandenseins der *NT5C2*-Genmutation umfasst, und Anweisungen für die Verwendung des Kits, wobei * eine Mutation zu einem Stoppcodon darstellt.

7. Kit zur Verwendung nach Anspruch 6, ferner umfassend Mittel zum Erhalten einer biologischen Probe von einem Subjekt.

8. Kit zur Verwendung nach Anspruch 6, ferner umfassend eine Kontrollprobe.

9. Kit zur Verwendung nach Anspruch 6, das die Mittel zum Assayieren von SNPs umfasst, Allel-spezifische PCR ist, und das Kit einen oder mehrere der Folgenden umfasst: Allel-spezifischen Primer, Allel-spezifischen Blocker, eine Locus-spezifische TaqMan-Sonde und einen Locus-spezifischen Primer.

## Revendications

1. Analogue nucléosidique choisi parmi la 6-mercaptopurine, la 6-thioguanine et la nélarabine pour l'utilisation dans un procédé de traitement d'un sujet ayant une leucémie aigüe lymphoblastique, dans lequel:
(a) si le sujet ne présente pas la présence d'une mutation du gène *NT5C2* détectable qui conduit à une mutation de protéine associée choisie dans le groupe constitué par R238W, R238L, R291W, K359Q, S360P, R367Q, L375F, D407A, D407Y et Q523*, l'analogue nucléosidique est la 6-mercaptopurine ou la 6-thioguanine; et
(b) si le sujet présente la présence d'une mutation du gène *NT5C2* détectable qui conduit à une mutation de protéine associée choisie dans le groupe constitué par R238W, R238L, R291W, K359Q, S360P, R367Q, L375F, D407A, D407Y et Q523*, l'analogue nucléosidique est la nélarabine.

2. Analogue nucléosidique pour l'utilisation dans un procédé selon la revendication 1, dans lequel la présence de la mutation du gène *NT5C2* est détectable en utilisant un essai de réaction en chaîne par polymérase (PCR) spécifique d'allèle;
dans lequel * représente une mutation à un codon STOP.

3. Analogue nucléosidique choisi parmi la 6-mercaptopurine, la 6-thioguanine et la nélarabine pour l'utilisation dans un procédé selon la revendication 1, dans lequel la présence de la mutation du gène *NT5C2* est détectable en effectuant au moins l'un parmi un séquençage d'ADN radioactif ou fluorescent, une réaction en chaîne par polymérase (PCR), une transcription inverse PCR (RTPCR), un clivage par endonucléase de restriction spécifique d'allèle, une détection de mésappariement-réparation, une liaison de protéine MutS, une électrophorèse sur gel en gradient dénaturant, une détection de polymorphisme de conformation simple brin, un clivage par RNase à des paires de bases mésappariées, un clivage chimique ou enzymatique d'un ADN hétéroduplex, les procédés reposant sur une extension d'amorce spécifique d'oligonucléotide, une analyse des bits génétiques, un essai de ligature des oligonucléotides, une réaction en chaîne par ligature (LCR) spécifique d'oligonucléotide, une gap-LCR, les essais d'acides nucléiques peptidiques (PNA), les analyses Southern Blot, les analyses de polymorphisme de conformation des simples brins (SSCP), un séquençage d'acide désoxyribonucléique, une analyse de polymorphisme de longueur des fragments de restriction (RFLP).

4. Analogue nucléosidique choisi parmi la 6-mercaptopurine, la 6-thioguanine et la nélarabine pour l'utilisation dans un procédé selon la revendication 1, dans lequel la présence de la mutation du gène *NT5C2* est détectable en utilisant un procédé du groupe comprenant une analyse Northern blot, les essais de protection contre les nucléases (NPA), une hybridation *in situ,* et un séquençage d'ARNm par transcription inverse-réaction en chaîne par polymérase (RT-PCR), ou dans lequel la détection du biomarqueur choisi parmi la protéine codée par la mutation du gène *NT5C2* comprend l'utilisation d'un procédé du groupe comprenant une immunocoloration, une immunoprécipitation, une immunoélectrophorèse, une immunoempreinte, un essai BCA pour quantifier les concentrations de protéine, un Western blot, une spectrophotométrie, et les essais enzymatiques.

5. Analogue nucléosidique choisi parmi la 6-mercaptopurine, la 6-thioguanine et la nélarabine pour l'utilisation dans un procédé selon la revendication 1, dans lequel la présence de la mutation du gène *NT5C2* est détectable dans un échantillon biologique du sujet qui est choisi dans le groupe constitué par le sérum, le sang, le sang périphérique, le plasma, les cellules sanguines, un épanchement pleural, le liquide céphalo-rachidien, la moelle osseuse ou une biopsie tissulaire.

6. Trousse pour l'utilisation dans la détection dans un échantillon biologique de la présence d'une mutation du gène *NT5C2* détectable qui conduit à une mutation de protéine associée choisie dans le groupe constitué par R238W, R238L, R291W, K359Q, S360P, R367Q, L375F, D407A, D407Y et Q523*, la trousse comprenant des réactifs pour détecter la présence de ladite mutation du gène *NT5C2,* et des instructions pour utiliser la trousse, où * représente une mutation à un codon STOP.

7. Trousse pour l'utilisation selon la revendication 6, comprenant en outre des moyens pour obtenir un échantillon biologique auprès d'un sujet.

8. Trousse pour l'utilisation selon la revendication 6, comprenant en outre un échantillon témoin.

9. Trousse pour l'utilisation selon la revendication 6, qui comprend le moyen pour tester des SNP est une PCR spécifique d'allèle et la trousse comprend un ou plusieurs des suivants: amorce spécifique d'allèle, bloqueur spécifique d'allèle, sonde TaqMan spécifique de locus, et une amorce spécifique de locus.


### SEQUENCE LISTING

<110> FERRANDO, Adolfo RABADAN, Raul PALOMERO, Teresa
<120> TECHNIQUES FOR TREATING ACUTE LYMPHOBLASTIC LEUKEMIA (ALL)
<130> P5180PC00
<160> 9
<170> PatentIn version 3.5
<210> 1
   <211> 13
   <212> DNA
   <213> Homo sapiens
<400> 1
   ggtggcgaac ttt 13
<210> 2
   <211> 13
   <212> DNA
   <213> Homo sapiens
<220>
   <221> misc_feature
   <222> (7)..(7)
   <223> n is g or a
<400> 2
   ggtggcnaac ttt 13
<210> 3
   <211> 13
   <212> DNA
   <213> Homo sapiens
<400> 3
   attttaaaat caa 13
<210> 4
   <211> 13
   <212> DNA
   <213> Homo sapiens
<220>
   <221> misc_feature
   <222> (7)..(7)
   <223> n is a or c
<400> 4
   attttanaat caa 13
<210> 5
   <211> 13
   <212> DNA
   <213> Homo sapiens
<400> 5
   atcttgacag cag 13
<210> 6
   <211> 13
   <212> DNA
   <213> Homo sapiens
<220>
   <221> misc_feature
   <222> (7)..(7)
   <223> n is a or c
<400> 6
   atcttgncag cag 13
<210> 7
   <211> 13
   <212> DNA
   <213> Homo sapiens
<400> 7
   cggcaccagc tga 13
<210> 8
   <211> 13
   <212> DNA
   <213> Homo sapiens
<220>
   <221> misc_feature
   <222> (7)..(7)
   <223> n is c or t
<400> 8
   cggcacnagc tga 13
<210> 9
   <211> 401
   <212> DNA
   <213> Homo sapiens
<220>
   <221> misc_feature
   <222> (201)..(201)
   <223> n is g or a
<400> 9
   atgaatcata gtatttaatg gttaaatact attggaatgc tgattttaat atagggacag      60
   ttttctcatt gttcactctg ttggttcagg ttcttctgat acgatctgtg acctgttggg     120
   agccaaggga aaagacattt tgtatattgg agatcacatt tttggggaca ttttaaaatc     180
   aaagaaacgg caagggtggc naactttttt ggtgattcct gaactcgcac aggagctaca     240
   tgtctggact gacaagagtt gtaagccatt cataaatctt tggtgtaatt tatatctaaa     300
   tgaccagctt tgaatcagtc gtctttgata aaccattact ttgtattcag gaagtcagag     360
   gtggtcaaaa ggcaggcctt gatatttgga tacttccttc c                         401